# EUROPEAN PATENT APPLICATION

(11) **EP 4 830 767 A2**
(43) Date of publication of application: **28.10.2026**
(21) Application number: 26196205.4
(22) Date of filing: 22.03.2019
(51) Int. Cl.: A61N 1/368

(54) **AV SYNCHRONOUS VFA CARDIAC THERAPY**

(30) Priority: 23.03.2018 US 201862647426 P
(62) Document number(s) of the earlier application(s) in accordance with Art. 76 EPC: 19715756.3 / 3 768 369
(71) Applicant: Medtronic, Inc., Minneapolis, Minnesota 55432 (US)
(72) Inventor: YANG, Zhongping, Minneapolis (US); ANDERSON, Thomas, A., Minneapolis (US); COLIN, Brian, P., Minneapolis (US); CLEMENS, William J., Minneapolis (US); GHOSH, Subham, Minneapolis (US); GILLBERG, Jeffrey M., Minneapolis (US); VERBEEK, Maurice T.I., Minneapolis (US); CAMPS, Toine, Minneapolis (US); KORNET, Lilian, Minneapolis (US); STEGEMANN, Berthold, Minneapolis (US); RUTTEN, Jean, Minneapolis (US)
(74) Representative: Zimmermann & Partner Patentanwälte mbB

(57) **Abstract**

VfA cardiac therapy uses an implantable medical device or system. The implantable medical device includes a tissue-piercing electrode implanted in the basal and/or septal region of the left ventricular myocardium of the patient's heart from the triangle of Koch region of the right atrium through the right atrial endocardium and central fibrous body. The device may include a right atrial electrode, a right atrial motion detector, or both. The device may be implanted completely within the patient's heart or may use one or more leads to implant electrodes in the patient's heart. The device may be used to provide cardiac therapy, including single or multiple chamber pacing, atrioventricular synchronous pacing, asynchronous pacing, triggered pacing, cardiac resynchronization pacing, or tachycardia-related therapy. A separate medical device may be used to provide some functionality for cardiac therapy, such as sensing, pacing, or shock therapy.

## Description

The present application claims the benefit of U.S. Provisional Application Serial No. 62/647,426, filed March 23, 2018, which is incorporated herein by reference in its entirety.

The present disclosure relates to implantable medical devices, systems, and methods. In particular, the present disclosure relates to implantable medical devices, systems, and methods for ventricle-from-atrium (VfA) cardiac therapy, including single chamber or multiple chamber pacing (e.g., dual or triple chamber pacing), atrioventricular synchronous pacing, asynchronous pacing, triggered pacing, cardiac resynchronization pacing, or tachycardia-related therapy.

The cardiac conduction system includes the sinus atrial (SA) node, the atrioventricular (AV) node, the bundle of His, bundle branches and Purkinje fibers. A heart beat is initiated in the SA node, which may be described as the natural "pacemaker" of the heart. An electrical impulse arising from the SA node causes the atrial myocardium to contract. The signal is conducted to the ventricles via the AV node which inherently delays the conduction to allow the atria to stop contracting before the ventricles begin contracting thereby providing proper AV synchrony. The electrical impulse is conducted from the AV node to the ventricular myocardium via the bundle of His, bundle branches, and Purkinje fibers.

Patients with a conduction system abnormality, such as poor AV node conduction or poor SA node function, may receive an implantable medical device (IMD), such as a pacemaker, to restore a more normal heart rhythm and AV synchrony. Some types of IMDs, such as cardiac pacemakers, implantable cardioverter defibrillators (ICDs), or cardiac resynchronization therapy (CRT) devices, provide therapeutic electrical stimulation to a heart of a patient via electrodes on one or more implantable endocardial, epicardial, or coronary venous leads that are positioned in or adjacent to the heart. The therapeutic electrical stimulation may be delivered to the heart in the form of pulses or shocks for pacing, cardioversion, or defibrillation. In some cases, an IMD may sense intrinsic depolarizations of the heart, and control the delivery of therapeutic stimulation to the heart based on the sensing.

Delivery of therapeutic electrical stimulation to the heart can be useful in addressing cardiac conditions such as ventricular dyssynchrony that may occur in patients. Ventricular dyssynchrony may be described as a lack of synchrony or a difference in the timing of contractions in different ventricles of the heart. Significant differences in timing of contractions can reduce cardiac efficiency. CRT, delivered by an IMD to the heart, may enhance cardiac output by resynchronizing the electromechanical activity of the ventricles of the heart. CRT is sometimes referred to as "triple chamber pacing" because of pacing the right atrium, right ventricle, and left ventricle.

Cardiac arrhythmias may be treated by delivering electrical shock therapy for cardioverting or defibrillating the heart in addition to cardiac pacing, for example, from an ICD, which may sense a patient's heart rhythm and classify the rhythm according to an arrhythmia detection scheme in order to detect episodes of tachycardia or fibrillation. Arrhythmias detected may include ventricular tachycardia (VT), fast ventricular tachycardia (FVT), ventricular fibrillation (VF), atrial tachycardia (AT) and atrial fibrillation (AT). Anti-tachycardia pacing (ATP), a painless therapy, can be used to treat ventricular tachycardia (VT) to substantially terminate many monomorphic fast rhythms. While ATP is painless, ATP may not deliver effective therapy for all types of VTs. For example, ATP may not be as effective for polymorphic VTs, which has variable morphologies. Polymorphic VTs and ventricular fibrillation (VFs) can be more lethal and may require expeditious treatment by shock.

Dual chamber medical devices are available that include a transvenous atrial lead carrying electrodes that may be placed in the right atrium and a transvenous ventricular lead carrying electrodes that may be placed in the right ventricle via the right atrium. The dual chamber medical device itself is generally implanted in a subcutaneous pocket and the transvenous leads are tunneled to the subcutaneous pocket. A dual chamber medical device may sense atrial electrical signals and ventricular electrical signals and can provide both atrial pacing and ventricular pacing as needed to promote a normal heart rhythm and AV synchrony. Some dual chamber medical devices can treat both atrial and ventricular arrhythmias.

Intracardiac medical devices, such as a leadless pacemaker, have been introduced or proposed for implantation entirely within a patient's heart, eliminating the need for transvenous leads. A leadless pacemaker may include one or more electrodes on its outer housing to deliver therapeutic electrical signals and/or sense intrinsic depolarizations of the heart. Intracardiac medical devices may provide cardiac therapy functionality, such as sensing and pacing, within a single chamber of the patient's heart. Single chamber intracardiac devices may also treat either atrial or ventricular arrhythmias or fibrillation. Some leadless pacemakers are not intracardiac and may be positioned outside of the heart and, in some examples, may be anchored to a wall of the heart via a fixation mechanism.

In some patients, single chamber devices may adequately address the patient's needs. However, single chamber devices capable of only single chamber sensing and therapy may not fully address cardiac conduction disease or abnormalities in all patients, for example, those with some forms of AV dyssynchrony or tachycardia. Dual chamber sensing and/or pacing functions, in addition to ICD functionality in some cases, may be used to restore more normal heart rhythms.

### SUMMARY

Various embodiments of the present disclosure relate to implantable medical devices, systems, and methods for VfA cardiac therapy, including single or multiple chamber pacing (e.g., dual or triple chamber pacing), atrioventricular synchronous pacing, asynchronous pacing, triggered pacing, cardiac resynchronization pacing, or tachycardia-related therapy. A tissue-piercing electrode is implanted in the basal and/or septal region of the left ventricular myocardium of the patient's heart from the triangle of Koch region of the right atrium through the right atrial endocardium and central fibrous body to facilitate the VfA cardiac therapy.

In one aspect, the present disclosure relates to an implantable medical device that includes a plurality of electrodes. The plurality of electrodes includes a tissue-piercing electrode implantable from the triangle of Koch region of the right atrium through the right atrial endocardium and central fibrous body to at least one of deliver cardiac therapy to and sense electrical activity of the left ventricle in the basal and/or septal region of the left ventricular myocardium of a patient's heart. The plurality of electrodes also includes a right atrial electrode positionable within the right atrium to at least one of deliver cardiac therapy and sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to monitor electrical activity of the right atrium using the right atrial electrode and deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity of the right atrium.

In another aspect, the present disclosure relates to a method that includes monitoring activity of the right atrium of a patient's heart using a right atrial electrode or a right atrial motion detector and delivering atrioventricular synchronous pacing using at least a tissue-piercing electrode implanted in the basal and/or septal region of the left ventricular myocardium of the patient's heart from the triangle of Koch region of the right atrium through the right atrial endocardium and central fibrous body including pacing one or both ventricles using at least the tissue-piercing electrode based on the monitored activity of the right atrium.

In another aspect, the present disclosure relates to an implantable medical device that includes a housing extending from a proximal end region to a distal end region. The device also includes a plurality of electrodes. The plurality of electrodes includes a tissue-piercing electrode leadlessly coupled to the distal end region of the housing and implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode leadlessly coupled to the housing and positionable within the right atrium to deliver cardiac therapy to or sense electrical activity of the right atrium of the patient's heart. The implantable medical device also includes a therapy delivery circuit within the housing operably coupled to the plurality of electrodes to deliver cardiac therapy to the patient's heart and a sensing circuit within the housing operably coupled to the plurality of electrodes to sense electrical activity of the patient's heart. The implantable medical device further includes a controller including processing circuitry within the housing operably coupled to the therapy delivery circuit and the sensing circuit. The controller is configured to monitor electrical activity of the right atrium using the right atrial electrode and deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity of the right atrium.

In another aspect, the present disclosure relates to an implantable medical device that includes a plurality of electrodes. The plurality of electrodes includes a tissue-piercing electrode implantable from the triangle of Koch region of the right atrium through the right atrial endocardium and central fibrous body to at least one of deliver cardiac therapy to and sense electrical activity of the left ventricle in the basal and/or septal region of the left ventricular myocardium of a patient's heart. The tissue-piercing electrode also includes a right atrial motion detector positionable within the right atrium to sense mechanical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to monitor mechanical activity of the right atrium using the right atrial motion detector and deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored mechanical activity of the right atrium.

In another aspect, the present disclosure relates to an implantable medical device that includes a plurality of electrodes. The plurality of electrodes includes a tissue-piercing electrode implantable from the triangle of Koch region of the right atrium through the right atrial endocardium and central fibrous body to at least one of deliver cardiac therapy to or sense electrical activity of the left ventricle in the basal and/or septal region of the left ventricular myocardium of a patient's heart. The plurality of electrodes also includes a right atrial electrode positionable within the right atrium to at least one of deliver cardiac therapy to or sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to monitor at least one of electrical activity of the right atrium using the right atrial electrode and electrical activity of the left ventricle using the tissue-piercing electrode implanted in the basal and/or septal region of the left ventricular myocardium of the patient's heart from the triangle of Koch region of the right atrium through the right atrial endocardium and central fibrous body. The controller is also configured to deliver cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity.

In another aspect, the present disclosure relates to a method that includes monitoring at least one of electrical activity of the right atrium of a patient's heart using a right atrial electrode and electrical activity of the left ventricle using a tissue-piercing electrode implanted in the basal and/or septal region of the left ventricular myocardium of the patient's heart from the triangle of Koch region of the right atrium through the right atrial endocardium and central fibrous body. The method also includes delivering cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body including pacing one or both ventricles based on the monitored electrical activity.

In another aspect, the present disclosure relates to an implantable medical device that includes a housing extending from a proximal end region to a distal end region. The implantable medical device also includes a plurality of electrodes. The plurality of electrodes includes a tissue-piercing electrode leadlessly coupled to the distal end region of the housing and implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode leadlessly coupled to the housing and positionable within the right atrium to deliver cardiac therapy to or sense electrical activity of the right atrium of the patient's heart. The implantable medical device also includes a therapy delivery circuit within the housing operably coupled to the plurality of electrodes to deliver cardiac therapy to the patient's heart and a sensing circuit within the housing operably coupled to the plurality of electrodes to sense electrical activity of the patient's heart. The implantable medical device further includes a controller including processing circuitry within the housing operably coupled to the therapy delivery circuit and the sensing circuit. The controller is configured to monitor at least one of electrical activity of the right atrium using the right atrial electrode and electrical activity of the left ventricle using the tissue-piercing electrode in the basal and/or septal region of the left ventricular myocardium of the patient's heart from the triangle of Koch region of the right atrium through the right atrial endocardium and central fibrous body. The controller is further configured to deliver cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity.

In another aspect, the present disclosure relates to an implantable medical device that includes a plurality of electrodes. The plurality of electrodes includes a tissue-piercing electrode implantable from the triangle of Koch region of the right atrium through the right atrial endocardium and central fibrous body to at least one of deliver cardiac therapy to and sense electrical activity of the left ventricle in the basal and/or septal region of the left ventricular myocardium of a patient's heart. The plurality of electrodes also includes a right atrial motion detector positionable within the right atrium to sense mechanical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to monitor mechanical activity of the right atrium using the right atrial motion detector and deliver cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored mechanical activity of the right atrium.

In another aspect, the present disclosure relates to an implantable medical device that includes a plurality of electrodes. The plurality of electrodes includes a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode positionable within the right atrium to deliver cardiac therapy to or sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to: monitor electrical activity of the right atrium using the right atrial electrode; monitor electrical activity of the left ventricle using the tissue-piercing electrode implanted in the basal and/or septal region of the left ventricular myocardium of the patient's heart from the triangle of Koch region of the right atrium through the right atrial endocardium and central fibrous body to sense electrical activity of the left ventricle in the basal and/or septal region of the left ventricular myocardium; and deliver tachycardia therapy based on the monitored electrical activities of the right atrium and the left ventricle.

In another aspect, the present disclosure relates to a method that includes monitoring electrical activity of the right atrium of a patient's heart using a right atrial electrode; monitoring electrical activity of the left ventricle of the patient's heart using a tissue-piercing electrode implanted in the basal and/or septal region of the left ventricular myocardium of the patient's heart from the triangle of Koch region of the right atrium through the right atrial endocardium and central fibrous body to sense electrical activity of the left ventricle in the basal and/or septal region of the left ventricular myocardium; and deliver tachycardia therapy based on the monitored electrical activities of the right atrium and the left ventricle.

In another aspect, the present disclosure relates to an implantable medical device that includes a plurality of electrodes. The plurality of electrodes includes a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode positionable within the right atrium to deliver cardiac therapy to or sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to: monitor electrical activity of the right atrium using the right atrial electrode; monitor electrical activity of the left ventricle using the tissue-piercing electrode implanted to sense electrical activity of the left ventricle in the basal and/or septal region of the left ventricular myocardium of the patient's heart from the triangle of Koch region of the right atrium through the right atrial endocardium and central fibrous body; and determine tachycardia of the patient's heart based on the monitored electrical activities of the right atrium and the left ventricle.

In another aspect, the present disclosure relates to an implantable medical device that includes a plurality of electrodes. The plurality of electrodes includes a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode positionable within the right atrium to deliver cardiac therapy to or sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to deliver at least one of anti-tachycardia pacing therapy using the plurality of electrodes and shock therapy using a separate medical device.

The above summary is not intended to describe each embodiment or every implementation of the present disclosure. A more complete understanding will become apparent and appreciated by referring to the following detailed description and claims taken in conjunction with the accompanying drawings. In other words, these and various other features and advantages will be apparent from a reading of the following detailed description.

### BRIEF DESCRIPTION OF THE DRAWINGS

**FIG. 1** is a conceptual diagram of an illustrative cardiac therapy system including an intracardiac medical device implanted in a patient's heart shown in a cross-sectional view and a separate medical device positioned outside of the patient's heart.
**FIG. 2** is an enlarged conceptual diagram of the intracardiac medical device of **FIG. 1** and anatomical structures of the patient's heart.
**FIG. 3** is a perspective view of the intracardiac medical device of **FIGS. 1-2** having a distal fixation and electrode assembly that includes a distal housing-based electrode implemented as a ring electrode.
**FIG. 4** is a block diagram of illustrative circuitry that may be enclosed within a housing of the intracardiac medical device of **FIGS. 1-3****,** for example, to provide the functionality and therapy described herein.
**FIG. 5** is a perspective view of another illustrative intracardiac medical device for use with, e.g., the illustrative system and device of **FIGS. 1-4****.**
**FIG. 6** is a flowchart of an illustrative cardiac therapy method for use with, e.g., the illustrative system and devices of **FIGS. 1-5****.**
**FIG. 7** is a flowchart of an illustrative pacing therapy method for use with, e.g., the illustrative method of **FIG. 6****.**
**FIG. 8** is a flowchart of another illustrative pacing therapy method for use with, e.g., the illustrative method of **FIG. 6****.**
**FIG. 9** is a flowchart of an illustrative tachycardia-related method for use with, e.g., the illustrative system and devices of **FIGS. 1-5****.**
**FIG. 10** is a flowchart of an illustrative tachycardia determination method for use with, e.g., the illustrative method of **FIG. 9****.**
**FIG. 11** is a flowchart of an illustrative tachycardia therapy determination method for use with, e.g., the illustrative method of **FIG. 10****.**
**FIG. 12** is a flowchart of another illustrative anti-tachycardia method for use with, e.g., the illustrative system and devices of **FIGS. 1-5****.**
**FIG. 13** is a flowchart of an illustrative tachycardia therapy delivery method for use with, e.g., the illustrative method of **FIG. 12****.**
**FIG. 14** is a flowchart of an illustrative shock therapy method for use with, e.g., the illustrative method of **FIG. 13****.**
**FIG. 15** is a conceptual diagram of a map of a patient's heart in a standard 17 segment view of the left ventricle showing various electrode implantation locations for use with, e.g., the exemplary system and devices of **FIGS. 1-5****.**
**FIGS. 16-18** are conceptual diagrams of illustrative cardiac therapy systems including medical devices including leads with electrodes implanted in a patient's heart shown in a cross-sectional view for use with, e.g., the illustrative methods of **FIGS. 6-14** or the illustrative system and devices of **FIGS. 1-5****.**
**FIG. 19** is a state diagram showing different illustrative modes for use with, e.g., the illustrative systems and devices of **FIGS. 1-5** and **FIGS. 16-18****.**
**FIG. 20** is a diagram of an illustrative system including electrode apparatus, display apparatus, and computing apparatus for use with, e.g., the illustrative systems and devices of **FIGS. 1-5** and **FIGS. 16-18****.**
**FIGS. 21-22** are diagrams of illustrative external electrode apparatus for measuring torso-surface potentials for use with, e.g., the illustrative systems and devices of **FIGS. 1-5** and **FIGS. 16-18****.**
**FIG. 23** is a flowchart of an illustrative method of detecting atrial activity using an atrial motion detector for use with, e.g., the illustrative systems and devices of **FIGS. 1-5** and **FIGS. 16-18****.**

### DETAILED DESCRIPTION

This disclosure relates to implantable medical devices, systems, and methods for VfA cardiac therapy, including single or multiple chamber pacing (e.g., dual or triple chamber pacing), atrioventricular synchronous pacing, asynchronous pacing, triggered pacing, cardiac resynchronization pacing, or tachycardia-related therapy. Although reference is made herein to implantable medical devices, such as a pacemaker or ICD, the methods and processes may be used with any medical devices, systems, or methods related to a patient's heart. Various other applications will become apparent to one of skill in the art having the benefit of the present disclosure.

It may be beneficial to provide an implantable medical device that is free of transvenous leads (e.g., a leadless device). It may also be beneficial to provide an implantable medical device capable of being used for various cardiac therapies, such as single or multiple chamber pacing (e.g., dual or triple chamber pacing), atrioventricular synchronous pacing, asynchronous pacing, triggered pacing, cardiac resynchronization pacing, or tachycardia-related therapy. Further, it may be beneficial to provide a system capable of communicating with a separate medical device, for example, to provide triggered pacing or to provide shock therapy in certain cases of tachycardia.

The present disclosure provides an implantable medical device including a tissue-piercing electrode and optionally a right atrial electrode and/or a right atrial motion detector. The tissue-piercing electrode may be implanted in the basal and/or septal region of the left ventricular myocardium of the patient's heart from the triangle of Koch region of the right atrium through the right atrial endocardium and central fibrous body. In a leadless implantable medical device, the tissue-piercing electrode may leadlessly extend from a distal end region of a housing of the device, and the right atrial electrode may be leadlessly coupled to the housing (e.g., part of or positioned on the exterior of). The right atrial motion detector may be within the implantable medical device. In a leaded implantable medical device, one or more of the electrodes may be coupled to the housing using an implantable lead. When the device is implanted, the electrodes may be used to sense electrical activity in one or more atria and/or ventricles of a patient's heart. The motion detector may be used to sense mechanical activity in one or more atria and/or ventricles of the patient's heart. In particular, the activity of the right atrium and the left ventricle may be monitored and, optionally, the activity of the right ventricle may be monitored. The electrodes may be used to deliver cardiac therapy, such as single chamber pacing for atrial fibrillation, atrioventricular synchronous pacing for bradycardia, asynchronous pacing, triggered pacing, cardiac resynchronization pacing for ventricular dyssynchrony, anti-tachycardia pacing, or shock therapy. Shock therapy may be initiated by the implantable medical device. A separate medical device, such as an extravascular ICD, which may also be implanted, may be in operative communication with the implantable medical device and may deliver an electrical shock in response to a trigger, such as a signaling pulse (e.g., triggering, signaling, or distinctive electrical pulse) provided by the device.

Reference will now be made to the drawings, which depict one or more aspects described in this disclosure. However, it will be understood that other aspects not depicted in the drawings fall within the scope of this disclosure. Like numbers used in the figures refer to like components, steps, and the like. However, it will be understood that the use of a reference character to refer to an element in a given figure is not intended to limit the element in another figure labeled with the same reference character. In addition, the use of different reference characters to refer to elements in different figures is not intended to indicate that the differently referenced elements cannot be the same or similar.

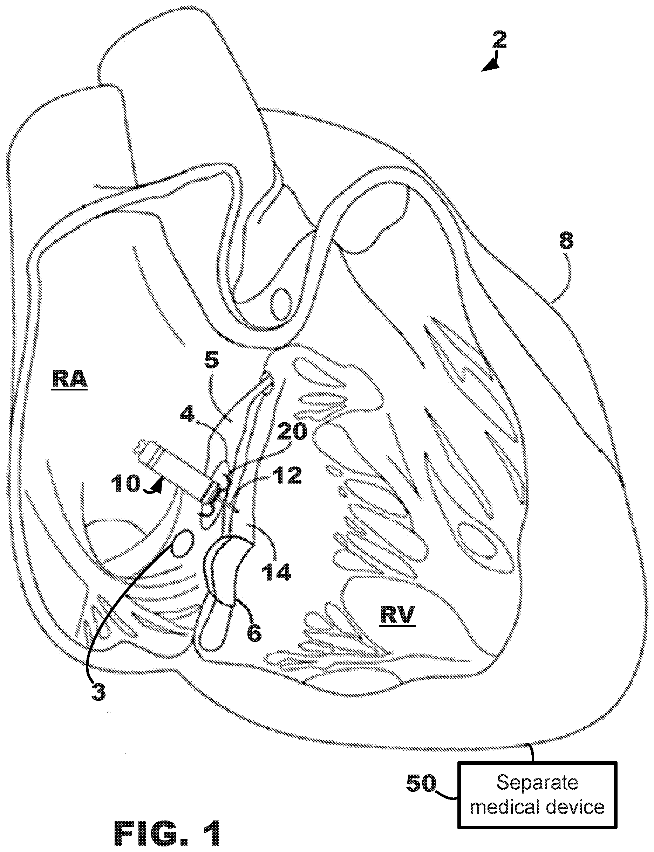

Although the present disclosure describes leadless and leaded implantable medical devices, reference is first made to **FIG. 1** showing a conceptual diagram of a cardiac therapy system **2** including an intracardiac medical device **10** that may be configured for single or dual chamber therapy and implanted in a patient's heart **8.** In some embodiments, the device **10** may be configured for single chamber pacing and may, for example, switch between single chamber and multiple chamber pacing (e.g., dual or triple chamber pacing). As used herein, "intracardiac" refers to a device configured to be implanted entirely within a patient's heart, for example, to provide cardiac therapy. The device **10** is shown implanted in the right atrium (RA) of the patient's heart **8** in a target implant region **4.** The device **10** may include one or more fixation members **20** that anchor a distal end of the device against the atrial endocardium in a target implant region **4.** The target implant region **4** may lie between the Bundle of His **5** and the coronary sinus **3** and may be adjacent the tricuspid valve **6.** The device **10** may be described as a ventricle-from-atrium (VfA) device, which may sense or provide therapy to one or both ventricles (e.g., right ventricle, left ventricle, or both ventricles, depending on the circumstances) while being generally disposed in the right atrium. In particular, the device **10** may include a tissue-piercing electrode that may be implanted in the basal and/or septal region of the left ventricular myocardium of the patient's heart from the triangle of Koch region of the right atrium through the right atrial endocardium and central fibrous body.

The device **10** may be described as a leadless implantable medical device. As used herein, "leadless" refers to a device being free of a lead extending out of the patient's heart **8.** In other words, a leadless device may have a lead that does not extend from outside of the patient's heart to inside of the patient's heart. Some leadless devices may be introduced through a vein, but once implanted, the device is free of, or may not include, any transvenous lead and may be configured to provide cardiac therapy without using any transvenous lead. A leadless VfA device, in particular, does not use a lead to operably connect to an electrode in the ventricle when a housing of the device is positioned in the atrium. A leadless electrode may be coupled to the housing of the medical device without using a lead between the electrode and the housing.

The device **10** may include one or more dart electrodes **12** having a straight shaft extending from the distal end region of device **10,** through the atrial myocardium and the central fibrous body, and into the ventricular myocardium **14** or along the ventricular septum, without perforating entirely through the ventricular endocardial or epicardial surfaces. In other words, the dart electrode **12** may not pierce through the ventricular wall into the blood volume. The dart electrode **12** may carry an electrode at the distal end region of the shaft for positioning the electrode within the ventricular myocardium for sensing ventricular signals and delivering ventricular pulses (e.g., to depolarize the left ventricle to initiate a contraction of the left ventricle). In some examples, the electrode at the distal end region of the shaft is a cathode electrode provided for use in a bipolar electrode pair for pacing and sensing. While the implant region **4** is shown in **FIG. 1** to enable one or more electrodes of the one or more dart electrodes **12** to be positioned in the ventricular myocardium, it is recognized that a device having the aspects disclosed herein may be implanted at other locations for multiple chamber pacing (e.g., dual or triple chamber pacing), single chamber pacing with multiple chamber sensing, single chamber pacing and/or sensing, or other clinical therapy and applications as appropriate.

The cardiac therapy system **2** may also include a separate medical device **50** (depicted diagrammatically in **FIG. 1**), which may be positioned outside the patient's heart **8** (e.g., subcutaneously) and may be operably coupled to the patient's heart **8** to deliver cardiac therapy thereto. In one example, separate medical device **50** may be an extravascular ICD. In some embodiments, an extravascular ICD may include a defibrillation lead with a defibrillation electrode. A therapy vector may exist between the defibrillation electrode on the defibrillation lead and a housing electrode of the ICD. Further, one or more electrodes of the ICD may also be used for sensing electrical signals related to the patient's heart **8.** The ICD may be configured to deliver shock therapy including one or more defibrillation or cardioversion shocks. For example, if an arrhythmia is sensed, the ICD may send a pulse via the electrical lead wires to shock the heart and restore its normal rhythm. In some examples, the ICD may deliver shock therapy without placing electrical lead wires within the heart or attaching electrical wires directly to the heart (subcutaneous ICDs). Examples of extravascular, subcutaneous ICDs that may be used with the system **2** described herein may be described in U.S. Patent No. 9,278,229 (Reinke et al.), issued 8 March 2016, which is incorporated herein by reference in its entirety.

In the case of shock therapy, e.g., the defibrillation shocks provided by the defibrillation electrode of the defibrillation lead, separate medical device **50** (e.g., extravascular ICD) may include a control circuit that uses a therapy delivery circuit to generate defibrillation shocks having any of a number of waveform properties, including leading-edge voltage, tilt, delivered energy, pulse phases, and the like. The therapy delivery circuit may, for instance, generate monophasic, biphasic, or multiphasic waveforms. Additionally, the therapy delivery circuit may generate defibrillation waveforms having different amounts of energy. For example, the therapy delivery circuit may generate defibrillation waveforms that deliver a total of between approximately 60-80 Joules (J) of energy for subcutaneous defibrillation.

The separate medical device **50** may include a sensing circuit. The sensing circuit may be configured to obtain electrical signals sensed via one or more combinations of electrodes and process the obtained signals. The components of the sensing circuit may be analog components, digital components, or a combination thereof. The sensing circuit may, for example, include one or more sense amplifiers, filters, rectifiers, threshold detectors, analog-to-digital converters (ADCs) or the like. The sensing circuit may convert the sensed signals to digital form and provide the digital signals to the control circuit for processing or analysis. For example, the sensing circuit may amplify signals from sensing electrodes and convert the amplified signals to multibit digital signals by an ADC. The sensing circuit may also compare processed signals to a threshold to detect the existence of atrial or ventricular depolarizations (e.g., P- or R-waves) and indicate the existence of the atrial depolarization (e.g., P-waves) or ventricular depolarizations (e.g., R-waves) to the control circuit.

The device **10** and the separate medical device **50** may cooperate to provide cardiac therapy to the patient's heart **8.** For example, the device **10** and the separate medical device **50** may be used to detect tachycardia, monitor tachycardia, and/or provide tachycardia-related therapy. For example, the device **10** may communicate with the separate medical device **50** wirelessly to trigger shock therapy using the separate medical device **50.** As used herein, "wirelessly" refers to an operative coupling or connection without using a metal conductor between the device **10** and the separate medical device **50.** In one example, wireless communication may use a distinctive, signaling, or triggering electrical pulse provided by the device **10** that conducts through the patient's tissue and is detectable by the separate medical device **50.** In another example, wireless communication may use a communication interface (e.g., an antenna) of the device **10** to provide electromagnetic radiation that propagates through patient's tissue and is detectable, for example, using a communication interface (e.g., an antenna) of the separate medical device **50.**

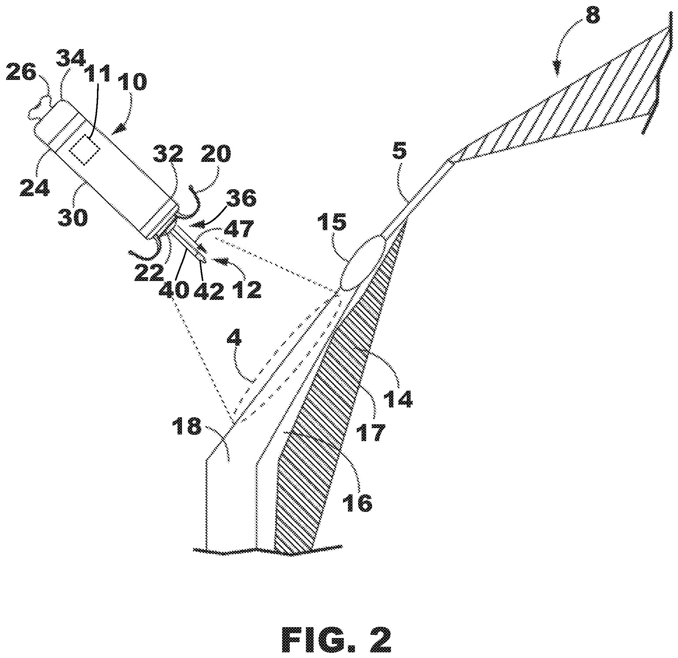

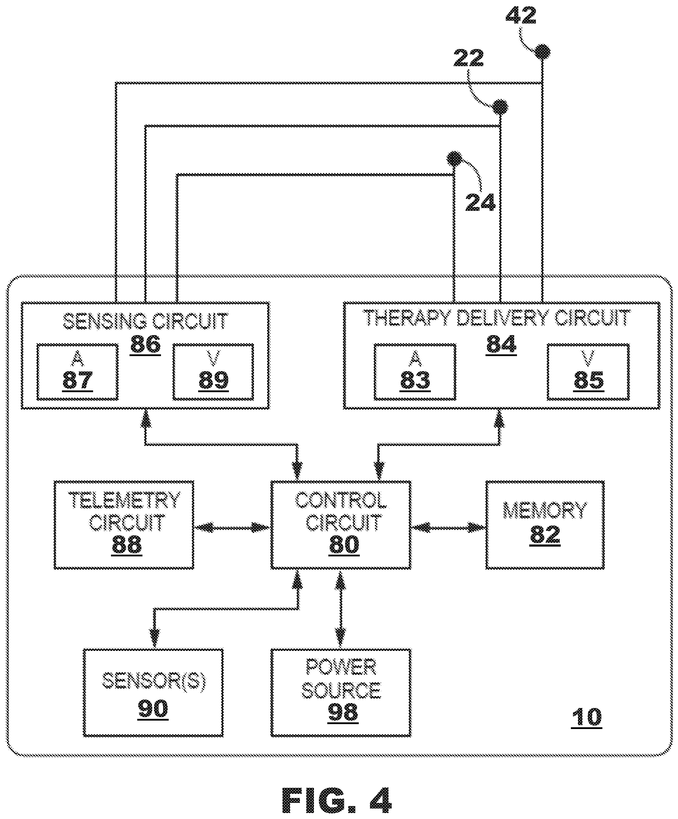

**FIG. 2** is an enlarged conceptual diagram of the intracardiac medical device **10** and anatomical structures of the patient's heart **8.** The intracardiac device **10** may include a housing **30.** The housing **30** may define a hermetically sealed internal cavity in which internal components of the device **10** reside, such as a sensing circuit, therapy delivery circuit, control circuit, memory, telemetry circuit, other optional sensors, and a power source as generally described in conjunction with **FIG. 4** below. The housing **30** may be formed from an electrically conductive material including titanium or titanium alloy, stainless steel, MP35N (a non-magnetic nickel-cobalt-chromium-molybdenum alloy), platinum alloy or other bio-compatible metal or metal alloy. In other examples, the housing **30** may be formed from a non-conductive material including ceramic, glass, sapphire, silicone, polyurethane, epoxy, acetyl co-polymer plastics, polyether ether ketone (PEEK), a liquid crystal polymer, or other biocompatible polymer.

The housing **30** may be described as extending between a distal end region **32** and a proximal end region **34** in a generally cylindrical shape to facilitate catheter delivery. In other embodiments, the housing **30** may be prismatic or any other shape so as to perform the functionality and utility described herein. The housing **30** may include a delivery tool interface member **26,** e.g., at the proximal end **34,** for engaging with a delivery tool during implantation of the device **10.**

All or a portion of the housing **30** may function as an electrode during cardiac therapy, for example, in sensing and/or pacing. In the example shown, the housing-based electrode **24** is shown to circumscribe a proximal portion of the housing **30.** When the housing **30** is formed from an electrically conductive material, such as a titanium alloy or other examples listed above, portions of the housing **30** may be electrically insulated by a non-conductive material, such as a coating of parylene, polyurethane, silicone, epoxy, or other biocompatible polymer, leaving one or more discrete areas of conductive material exposed to define the proximal housing-based electrode **24.** When the housing **30** is formed from a non-conductive material, such as a ceramic, glass or polymer material, an electrically-conductive coating or layer, such as a titanium, platinum, stainless steel, or alloys thereof, may be applied to one or more discrete areas of the housing **30** to form the proximal housing-based electrode **24.** In other examples, the proximal housing-based electrode **24** may be a component, such as a ring electrode, that is mounted or assembled onto the housing **30.** The proximal housing-based electrode **24** may be electrically coupled to internal circuitry of the device **10,** e.g., via the electrically-conductive housing **30** or an electrical conductor when the housing **30** is a non-conductive material.

In the example shown, the proximal housing-based electrode **24** is located nearer to the housing proximal end region **34** than the housing distal end region **32** and is therefore referred to as a "proximal housing-based electrode" **24.** In other examples, however, the housing-based electrode **24** may be located at other positions along the housing **30,** e.g., relatively more distally than the position shown.

At the distal end region **32,** the device **10** may include a distal fixation and electrode assembly **36,** which may include one or more fixation members **20,** in addition to one or more dart electrodes **12** of equal or unequal length. The dart electrode **12** may include a shaft **40** extending distally away from the housing distal end region **32** and may include one or more electrode elements, such as a tip electrode **42** at or near the free, distal end region of the shaft **40.** The tip electrode **42** may have a conical or hemispherical distal tip with a relatively narrow tip diameter (e.g., less than about 1 mm) for penetrating into and through tissue layers without using a sharpened tip or needle-like tip having sharpened or beveled edges.

The shaft **40** of the dart electrode **12** may be a normally straight member and may be rigid. In other embodiments, the shaft **40** may be described as being relatively stiff but still possessing limited flexibility in lateral directions. Further, the shaft **40** may be non-rigid to allow some lateral flexing with heart motion. However, in a relaxed state, when not subjected to any external forces, the shaft **40** may maintain a straight position as shown to hold the tip electrode **42** spaced apart from the housing distal end region **32** at least by the height **47** of the shaft **40.** The dart electrode **12** may be configured to pierce through one or more tissue layers to position the tip electrode **42** within a desired tissue layer, e.g., the ventricular myocardium. As such, the height **47** of the shaft **40** may correspond to the expected pacing site depth, and the shaft may have a relatively high compressive strength along its longitudinal axis to resist bending in a lateral or radial direction when pressed against the implant region **4.** If a second dart electrode **12** is employed, its length may be unequal to the expected pacing site depth and may be configured to act as an indifferent electrode for delivering of pacing energy to the tissue. A longitudinal axial force may be applied against the tip electrode **42,** e.g., by applying longitudinal pushing force to the proximal end **34** of the housing **30,** to advance the dart electrode **12** into the tissue within target implant region. The shaft **40** may be longitudinally non-compressive. The shaft **40** may be elastically deformable in lateral or radial directions when subjected to lateral or radial forces to allow temporary flexing, e.g., with tissue motion, but may return to its normally straight position when lateral forces diminish. When the shaft **40** is not exposed to any external force, or to only a force along its longitudinal central axis, the shaft **40** may retain a straight, linear position as shown.

The one or more fixation members **20** may be described as one or more "tines" having a normally curved position. The tines may be held in a distally extended position within a delivery tool. The distal tips of tines may penetrate the heart tissue to a limited depth before elastically curving back proximally into the normally curved position (shown) upon release from the delivery tool. Further, the fixation members **20** may include one or more aspects described in, for example, U.S. Patent No. 9,675,579 (Grubac et al.), issued 13 June 2017, and U.S. Patent No. 9,119,959 (Rys et al.), issued 1 September 2015, each of which is incorporated herein by reference in its entirety.

In some examples, the distal fixation and electrode assembly **36** includes a distal housing-based electrode **22.** In the case of using the device **10** as a pacemaker for multiple chamber pacing (e.g., dual or triple chamber pacing) and sensing, the tip electrode **42** may be used as a cathode electrode paired with the proximal housing-based electrode **24** serving as a return anode electrode. Alternatively, the distal housing-based electrode **22** may serve as a return anode electrode paired with tip electrode **42** for sensing ventricular signals and delivering ventricular pacing pulses. In other examples, the distal housing-based electrode **22** may be a cathode electrode for sensing atrial signals and delivering pacing pulses to the atrial myocardium in the target implant region **4.** When the distal housing-based electrode **22** serves as an atrial cathode electrode, the proximal housing-based electrode **24** may serve as the return anode paired with the tip electrode **42** for ventricular pacing and sensing and as the return anode paired with the distal housing-based electrode **22** for atrial pacing and sensing.

As shown in this illustration, the target implant region **4** in some pacing applications is along the atrial endocardium **18,** generally inferior to the AV node **15** and the His bundle **5.** The dart electrode **42** may define the height **47** of the shaft **40** for penetrating through the atrial endocardium **18** in the target implant region **4,** through the central fibrous body **16,** and into the ventricular myocardium **14** without perforating through the ventricular endocardial surface **17.** When the height **47** of the dart electrode **12** is fully advanced into the target implant region **4,** the tip electrode **42** may rest within the ventricular myocardium **14,** and the distal housing-based electrode **22** may be positioned in intimate contact with or close proximity to the atrial endocardium **18.** The dart electrode **12** may have a total combined height **47** of tip electrode **42** and shaft **40** from about 3 mm to about 8 mm in various examples. The diameter of the shaft **40** may be less than about 2 mm, and may be about 1 mm or less, or even about 0.6 mm or less.

The device **10** may include a motion detector **11** within the housing **30.** The motion detector **11** may be used to monitor mechanical activity, such as atrial mechanical activity (e.g., an atrial contraction) and/or ventricular mechanical activity (e.g., a ventricular contraction). In some embodiments, the motion detector **11** may be used to detect right atrial mechanical activity. A non-limiting example of a motion detector **11** includes an accelerometer. In some embodiments, the mechanical activity detected by the motion detector **11** may be used to supplement or replace electrical activity detected by one or more of the electrodes of the device **10.** For example, the motion detector **11** may be used in addition to, or as an alternative to, the proximal housing-based electrode **24.**

The motion detector **11** may also be used for rate response detection or to provide a rate-responsive IMD. Various techniques related to rate response may be described in U.S. Patent No. 5,154,170 (Bennett et al.), issued October 13, 1992, entitled "Optimization for rate responsive cardiac pacemaker," and U.S. Patent No. 5,562,711 (Yerich et al.), issued October 8, 1996, entitled "Method and apparatus for rate-responsive cardiac pacing," each of which is incorporated herein by reference in its entirety.

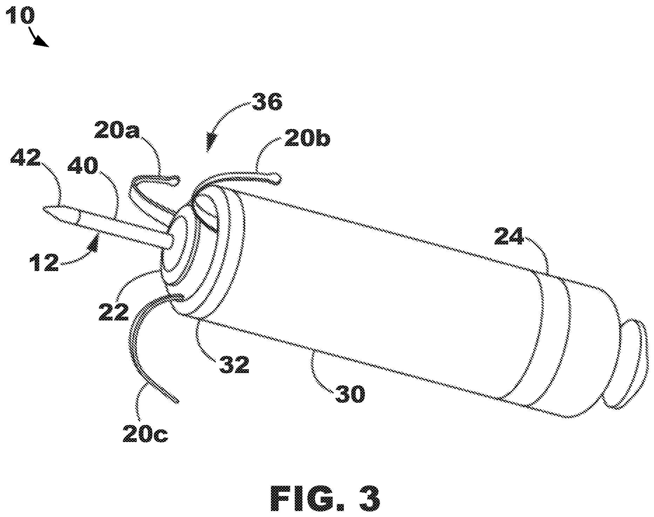

**FIG. 3** is a three-dimensional perspective view of the device **10** capable of cardiac therapy. As shown, the distal fixation and electrode assembly **36** includes the distal housing-based electrode **22** implemented as a ring electrode. The distal housing-based electrode **22** may be positioned in intimate contact with or operative proximity to atrial tissue when fixation member tines **20a, 20b** and **20c** of the fixation members **20,** engage with the atrial tissue. The tines **20a, 20b** and **20c,** which may be elastically deformable, may be extended distally during delivery of device **10** to the implant site. For example, the tines **20a, 20b,** and **20c** may pierce the atrial endocardial surface as the device **10** is advanced out of the delivery tool and flex back into their normally curved position (as shown) when no longer constrained within the delivery tool. As the tines **20a, 20b** and **20c** curve back into their normal position, the fixation member **20** may pull the distal fixation member and electrode assembly **36** toward the atrial endocardial surface. As the distal fixation member and electrode assembly **36** is pulled toward the atrial endocardium, the tip electrode **42** may be advanced through the atrial myocardium and the central fibrous body and into the ventricular myocardium. The distal housing-based electrode **22** may then be positioned against the atrial endocardial surface.

The distal housing-based electrode **22** may include a ring formed of an electrically conductive material, such as titanium, platinum, iridium, or alloys thereof. The distal housing-based electrode **22** may be a single, continuous ring electrode. In other examples, portions of the ring may be coated with an electrically insulating coating, e.g., parylene, polyurethane, silicone, epoxy, or other insulating coating, to reduce the electrically conductive surface area of the ring electrode. For instance, one or more sectors of the ring may be coated to separate two or more electrically conductive exposed surface areas of the distal housing-based electrode **22.** Reducing the electrically conductive surface area of the distal housing-based electrode **22,** e.g., by covering portions of the electrically conductive ring with an insulating coating, may increase the electrical impedance of the distal housing-based **22,** and thereby, reduce the current delivered during a pacing pulse that captures the myocardium, e.g., the atrial myocardial tissue. A lower current drain may conserve the power source, e.g., one or more rechargeable or non-rechargeable batteries, of the device **10.**

As described above, the distal housing-based electrode **22** may be configured as an atrial cathode electrode for delivering pacing pulses to the atrial tissue at the implant site in combination with the proximal housing-based electrode **24** as the return anode. The electrodes **22** and **24** may be used to sense atrial P-waves for use in controlling atrial pacing pulses (delivered in the absence of a sensed P-wave) and for controlling atrial-synchronized ventricular pacing pulses delivered using the tip electrode **42** as a cathode and the proximal housing-based electrode **24** as the return anode. In other examples, the distal housing-based electrode **22** may be used as a return anode in conjunction with the cathode tip electrode **42** for ventricular pacing and sensing.

**FIG. 4** is a block diagram of circuitry that may be enclosed within the housing **30** (**FIG. 3**) to provide the functions of cardiac therapy using the device **10** according to one example. The separate medical device **50** (**FIG. 1**) may include some or all the same components, which may be configured in a similar manner. The electronic circuitry enclosed within housing **30** may include software, firmware, and hardware that cooperatively monitor atrial and ventricular electrical cardiac signals, determine when a cardiac therapy is necessary, and/or deliver electrical pulses to the patient's heart according to programmed therapy mode and pulse control parameters. The electronic circuitry may include a control circuit **80** (e.g., including processing circuitry), a memory **82,** a therapy delivery circuit **84,** a sensing circuit **86,** and/or a telemetry circuit **88.** In some examples, the device **10** includes one or more sensors **90** for producing a signal that is correlated to a physiological function, state, or condition of the patient, such as a patient activity sensor, for use in determining a need for pacing therapy and/or controlling a pacing rate.

The power source **98** may provide power to the circuitry of the device **10** including each of the components **80, 82, 84, 86, 88,** and **90** as needed. The power source **98** may include one or more energy storage devices, such as one or more rechargeable or non-rechargeable batteries. The connections between the power source **98** and each of the components **80, 82, 84, 86, 88,** and **90** are to be understood from the general block diagram illustrated but are not shown for the sake of clarity. For example, the power source **98** may be coupled to one or more charging circuits included in the therapy delivery circuit **84** for providing the power needed to charge holding capacitors included in the therapy delivery circuit **84** that are discharged at appropriate times under the control of the control circuit **80** for delivering pacing pulses, e.g., according to a dual chamber pacing mode such as DDI(R). The power source **98** may also be coupled to components of the sensing circuit **86,** such as sense amplifiers, analog-to-digital converters, switching circuitry, etc., sensors **90,** the telemetry circuit **88,** and the memory **82** to provide power to the various circuits.

Any suitable technique may be used to recharge a rechargeable power source **98.** In some embodiments, the device **10** may include an antenna, inductive coils, or other inductive coupling structures configured to couple to another device, such as an external charger or programmer, to receive power *in situ.* Various examples of charging a leadless implantable medical device are described in U.S. Patent Pub. No. 2018/0212451 (Schmidt et al.), filed January 26, 2017, entitled "Recharge of Implanted Medical Devices," which is incorporated herein by reference in its entirety. The device **10** may also be configured to use various techniques to extend the life of the power source **98,** such as a low-power mode.

Various examples of power sources and techniques related to power sources may be used, such as those found in, for example, U.S. Patent No. 8,383,269 (Scott et al.), granted February 26, 2013, U.S. Patent No. 8,105,714 (Schmidt et al.), granted January 31, 2012, and U.S. Patent No. 7,635,541 (Scott et al.), granted December 22, 2009, each of which is incorporated herein by reference in its entirety.

The functional blocks shown represent functionality included in the device **10** and may include any discrete and/or integrated electronic circuit components that implement analog, and/or digital circuits capable of producing the functions attributed to the medical device **10** herein. The various components may include processing circuitry, such as an application specific integrated circuit (ASIC), an electronic circuit, a processor (shared, dedicated, or group), and memory that execute one or more software or firmware programs, a combinational logic circuit, state machine, or other suitable components or combinations of components that provide the described functionality. The particular form of software, hardware, and/or firmware employed to implement the functionality disclosed herein will be determined primarily by the particular system architecture employed in the medical device and by the particular detection and therapy delivery methodologies employed by the medical device. Providing software, hardware, and/or firmware to accomplish the described functionality in the context of any modern cardiac medical device system, given the disclosure herein, is within the abilities of one of skill in the art.

The memory **82** may include any volatile, non-volatile, magnetic, or electrical non-transitory computer readable storage media, such as random-access memory (RAM), read-only memory (ROM), non-volatile RAM (NVRAM), electrically-erasable programmable ROM (EEPROM), flash memory, or any other memory device. Furthermore, the memory **82** may include a non-transitory computer readable media storing instructions that, when executed by one or more processing circuits, cause the control circuit **80** and/or other processing circuitry to perform a single, dual, or triple chamber pacing (e.g., single or multiple chamber pacing) function or other sensing and therapy delivery functions attributed to the device **10.** The non-transitory computer-readable media storing the instructions may include any of the media listed above.

The control circuit **80** may communicate, e.g., via a data bus, with the therapy delivery circuit **84** and the sensing circuit **86** for sensing cardiac electrical signals and controlling delivery of cardiac electrical stimulation therapies in response to sensed cardiac events, e.g., P-waves and R-waves, or the absence thereof. The tip electrode **42,** the distal housing-based electrode **22,** and the proximal housing-based electrode **24** may be electrically coupled to the therapy delivery circuit **84** for delivering electrical stimulation pulses to the patient's heart and to the sensing circuit **86** and for sensing cardiac electrical signals.

The sensing circuit **86** may include an atrial (A) sensing channel **87** and a ventricular (V) sensing channel **89.** The distal housing-based electrode **22** and the proximal housing-based electrode **24** may be coupled to the atrial sensing channel **87** for sensing atrial signals, e.g., P-waves attendant to the depolarization of the atrial myocardium. In examples that include two or more selectable distal housing-based electrodes, the sensing circuit **86** may include switching circuitry for selectively coupling one or more of the available distal housing-based electrodes to cardiac event detection circuitry included in the atrial sensing channel **87.** Switching circuitry may include a switch array, switch matrix, multiplexer, or any other type of switching device suitable to selectively couple components of the sensing circuit **86** to selected electrodes. The tip electrode **42** and the proximal housing-based electrode **24** may be coupled to the ventricular sensing channel **89** for sensing ventricular signals, e.g., R-waves attendant to the depolarization of the ventricular myocardium.

Each of the atrial sensing channel **87** and the ventricular sensing channel **89** may include cardiac event detection circuitry for detecting P-waves and R-waves, respectively, from the cardiac electrical signals received by the respective sensing channels. The cardiac event detection circuitry included in each of the channels **87** and **89** may be configured to amplify, filter, digitize, and rectify the cardiac electrical signal received from the selected electrodes to improve the signal quality for detecting cardiac electrical events. The cardiac event detection circuitry within each channel **87** and **89** may include one or more sense amplifiers, filters, rectifiers, threshold detectors, comparators, analog-to-digital converters (ADCs), timers, or other analog or digital components. A cardiac event sensing threshold, e.g., a P-wave sensing threshold and an R-wave sensing threshold, may be automatically adjusted by each respective sensing channel **87** and **89** under the control of the control circuit **80,** e.g., based on timing intervals and sensing threshold values determined by the control circuit **80,** stored in the memory **82,** and/or controlled by hardware, firmware, and/or software of the control circuit **80** and/or the sensing circuit **86.**

Upon detecting a cardiac electrical event based on a sensing threshold crossing, the sensing circuit **86** may produce a sensed event signal that is passed to the control circuit **80.** For example, the atrial sensing channel **87** may produce a P-wave sensed event signal in response to a P-wave sensing threshold crossing. The ventricular sensing channel **89** may produce an R-wave sensed event signal in response to an R-wave sensing threshold crossing. The sensed event signals may be used by the control circuit **80** for setting pacing escape interval timers that control the basic time intervals used for scheduling cardiac pacing pulses. A sensed event signal may trigger or inhibit a pacing pulse depending on the particular programmed pacing mode. For example, a P-wave sensed event signal received from the atrial sensing channel **87** may cause the control circuit **80** to inhibit a scheduled atrial pacing pulse and schedule a ventricular pacing pulse at a programmed atrioventricular (AV) pacing interval. If an R-wave is sensed before the AV pacing interval expires, the ventricular pacing pulse may be inhibited. If the AV pacing interval expires before the control circuit **80** receives an R-wave sensed event signal from the ventricular sensing channel **89,** the control circuit **80** may use the therapy delivery circuit **84** to deliver the scheduled ventricular pacing pulse synchronized to the sensed P-wave.

In some examples, the device **10** may be configured to deliver a variety of pacing therapies including bradycardia pacing, cardiac resynchronization therapy, post-shock pacing, and/or tachycardia-related therapy, such as ATP, among others. For example, the device **10** may be configured to detect non-sinus tachycardia and deliver ATP. The control circuit **80** may determine cardiac event time intervals, e.g., PP intervals between consecutive P-wave sensed event signals received from the atrial sensing channel **87,** RR intervals between consecutive R-wave sensed event signals received from the ventricular sensing channel **89,** and P-R and/or R-P intervals received between P-wave sensed event signals and R-wave sensed event signals. These intervals may be compared to tachycardia detection intervals for detecting non-sinus tachycardia. Tachycardia may be detected in a given heart chamber based on a threshold number of tachycardia detection intervals being detected.

The therapy delivery circuit **84** may include atrial pacing circuit **83** and ventricular pacing circuit **85.** Each pacing circuit **83** and **85** may include charging circuitry, one or more charge storage devices such as one or more low voltage holding capacitors, an output capacitor, and/or switching circuitry that controls when the holding capacitor(s) are charged and discharged across the output capacitor to deliver a pacing pulse to the pacing electrode vector coupled to respective pacing circuits **83** or **85.** The tip electrode **42** and the proximal housing-based electrode **24** may be coupled to the ventricular pacing circuit **85** as a bipolar cathode and anode pair for delivering ventricular pacing pulses, e.g., upon expiration of an AV or VV pacing interval set by the control circuit **80** for providing atrial-synchronized ventricular pacing and a basic lower ventricular pacing rate.

The atrial pacing circuit **83** may be coupled to the distal housing-based electrode **22** and the proximal housing-based electrode **24** to deliver atrial pacing pulses. The control circuit **80** may set atrial pacing intervals according to a programmed lower pacing rate or a temporary lower rate set according to a rate-responsive sensor indicated pacing rate. Atrial pacing circuit may be controlled to deliver an atrial pacing pulse if the atrial pacing interval expires before a P-wave sensed event signal is received from the atrial sensing channel **87.** The control circuit **80** starts an AV pacing interval in response to a delivered atrial pacing pulse to provide synchronized multiple chamber pacing (e.g., dual or triple chamber pacing).

Charging of a holding capacitor of the atrial or ventricular pacing circuit **83** or **85** to a programmed pacing voltage amplitude and discharging of the capacitor for a programmed pacing pulse width may be performed by the therapy delivery circuit **84** according to control signals received from the control circuit **80.** For example, a pace timing circuit included in the control circuit **80** may include programmable digital counters set by a microprocessor of the control circuit **80** for controlling the basic pacing time intervals associated with various single chamber or multiple chamber pacing (e.g., dual or triple chamber pacing) modes or anti-tachycardia pacing sequences. The microprocessor of the control circuit **80** may also set the amplitude, pulse width, polarity, or other characteristics of the cardiac pacing pulses, which may be based on programmed values stored in the memory **82.**

The device **10** may include other sensors **90** for sensing signals from the patient for use in determining a need for and/or controlling electrical stimulation therapies delivered by the therapy delivery circuit **84.** In some examples, a sensor indicative of a need for increased cardiac output may include a patient activity sensor, such as an accelerometer. An increase in the metabolic demand of the patient due to increased activity as indicated by the patient activity sensor may be determined by the control circuit **80** for use in determining a sensor-indicated pacing rate.

Control parameters utilized by the control circuit **80** for sensing cardiac events and controlling pacing therapy delivery may be programmed into the memory **82** via the telemetry circuit **88,** which may also be described as a communication interface. The telemetry circuit **88** includes a transceiver and antenna for communicating with an external device such as a programmer or home monitor, using radio frequency communication or other communication protocols. The control circuit **80** may use the telemetry circuit **88** to receive downlink telemetry from and send uplink telemetry to the external device. **In** some cases, the telemetry circuit **88** may be used to transmit and receive communication signals to/from another medical device implanted in the patient.

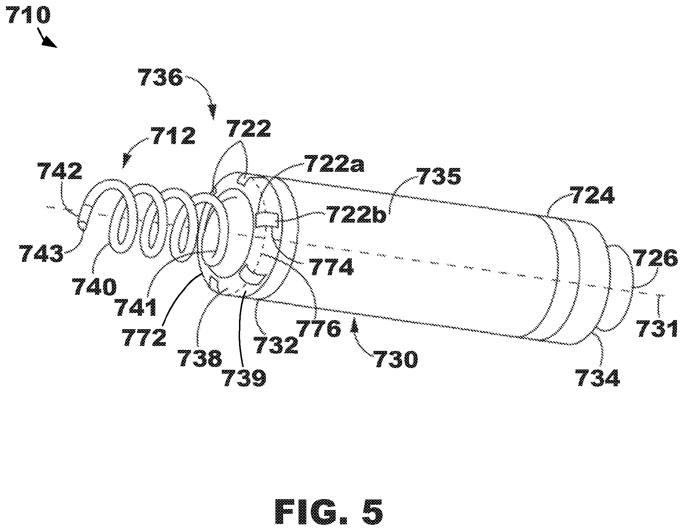

**FIG. 5** is a three-dimensional perspective view of another leadless intracardiac medical device **710** that may be configured for single or multiple chamber cardiac therapy (e.g., dual or triple chamber cardiac therapy) according to another example. The device **710** may include a housing **730** having an outer sidewall **735,** shown as a cylindrical outer sidewall, extending from a housing distal end region **732** to a housing proximal end region **734.** The housing **730** may enclose electronic circuitry configured to perform single or multiple chamber cardiac therapy, including atrial and ventricular cardiac electrical signal sensing and pacing the atrial and ventricular chambers. Delivery tool interface member **726** is shown on the housing proximal end region **734.**

A distal fixation and electrode assembly **736** may be coupled to the housing distal end region **732.** The distal fixation and electrode assembly **736** may include an electrically insulative distal member **772** coupled to the housing distal end region **732.** The tissue piercing electrode **712** extends away from the housing distal end region **732,** and multiple non-tissue piercing electrodes **722** may be coupled directly to the insulative distal member **772.** The tissue piercing electrode **712** extends in a longitudinal direction away from the housing distal end region **732** and may be coaxial with the longitudinal center axis **731** of the housing **730.**

The tissue piercing distal electrode **712** may include an electrically insulated shaft **740** and a tip electrode **742.** In some examples, the tissue piercing distal electrode **712** is an active fixation member including a helical shaft **740** and a distal cathode tip electrode **742.** The helical shaft **740** may extend from a shaft distal end region **743** to a shaft proximal end region **741,** which may be directly coupled to the insulative distal member **772.** The helical shaft **740** may be coated with an electrically insulating material, e.g., parylene or other examples listed herein, to avoid sensing or stimulation of cardiac tissue along the shaft length. The tip electrode **742** is at the shaft distal end region **743** and may serve as a cathode electrode for delivering ventricular pacing pulses and sensing ventricular electrical signals using the proximal housing-based electrode **724** as a return anode when the tip electrode **742** is advanced into ventricular tissue. The proximal housing-based electrode **724** may be a ring electrode circumscribing the housing **730** and may be defined by an uninsulated portion of the longitudinal sidewall **735.** Other portions of the housing **730** not serving as an electrode may be coated with an electrically insulating material as described above in conjunction with **FIG. 2****.**

Using two or more tissue-piercing electrodes (e.g., of any type) penetrating into the LV myocardium may be used for more localized pacing capture and may mitigate ventricular pacing spikes affecting capturing atrial tissue. In some embodiments, multiple tissue-piercing electrodes may include two or more of a dart-type electrode (e.g., electrode **12** of **FIGS. 1-2**), a helical-type electrode (e.g., electrode **712**) Non-limiting examples of multiple tissue-piercing electrodes include two dart electrodes, a helix electrode with a dart electrode extending therethrough (e.g., through the center), or dual intertwined helixes. Multiple tissue-piercing electrodes may also be used for bipolar or multi-polar pacing.

In some embodiments, one or more tissue-piercing electrodes (e.g., of any type) that penetrate into the LV myocardium may be a multi-polar tissue-piercing electrode. A multi-polar tissue-piercing electrode may include one or more electrically active and electrically separate elements, which may enable bipolar or multi-polar pacing from one or more tissue-piercing electrodes.

Multiple non-tissue piercing electrodes **722** may be provided along a periphery of the insulative distal member **772,** peripheral to the tissue piercing electrode **712.** The insulative distal member **772** may define a distal-facing surface **738** of the device **710** and a circumferential surface **739** that circumscribes the device **710** adjacent to the housing longitudinal sidewall **735.** Non-tissue piercing electrodes **722** may be formed of an electrically conductive material, such as titanium, platinum, iridium, or alloys thereof. In the illustrated embodiment, six non-tissue piercing electrodes **722** are spaced apart radially at equal distances along the outer periphery of insulative distal member **772,** however, two or more non-tissue piercing electrodes **722** may be provided.

Non-tissue piercing electrodes **722** may be discrete components each retained within a respective recess **774** in the insulative member **772** sized and shaped to mate with the non-tissue piercing electrode **722.** In other examples, the non-tissue piercing electrodes **722** may each be an uninsulated, exposed portion of a unitary member mounted within or on the insulative distal member **772.** Intervening portions of the unitary member not functioning as an electrode may be insulated by the insulative distal member **772** or, if exposed to the surrounding environment, may be coated with an electrically insulating coating, e.g., parylene, polyurethane, silicone, epoxy, or other insulating coating.

When the tissue piercing electrode **712** is advanced into cardiac tissue, at least one non-tissue piercing electrode **722** may be positioned against, in intimate contact with, or in operative proximity to, a cardiac tissue surface for delivering pulses and/or sensing cardiac electrical signals produced by the patient's heart. For example, non-tissue piercing electrodes **722** may be positioned in contact with right atrial endocardial tissue for pacing and sensing in the atrium when the tissue piercing electrode **712** is advanced into the atrial tissue and through the central fibrous body until the distal tip electrode **742** is positioned in direct contact with ventricular tissue, e.g., ventricular myocardium and/or a portion of the ventricular conduction system.

Non-tissue piercing electrodes **722** may be coupled to the therapy delivery circuit **84** and the sensing circuit **86** (see **FIG. 4**) enclosed by the housing **730** to function collectively as a cathode electrode for delivering atrial pacing pulses and for sensing atrial electrical signals, e.g., P-waves, in combination with the proximal housing-based electrode **724** as a return anode. Switching circuitry included in the sensing circuit **86** may be activated under the control of the control circuit **80** to couple one or more of the non-tissue piercing electrodes to the atrial sensing channel **87.** Distal, non-tissue piercing electrodes **722** may be electrically isolated from each other so that each individual one of the electrodes **722** may be individually selected by switching circuitry included in the therapy delivery circuit **84** to serve alone or in a combination of two or more of the electrodes **722** as an atrial cathode electrode. Switching circuitry included in the therapy delivery circuit **84** may be activated under the control of the control circuit **80** to couple one or more of the non-tissue piercing electrodes **722** to the atrial pacing circuit **83.** Two or more of the non-tissue piercing electrodes **722** may be selected at a time to operate as a multi-point atrial cathode electrode.

Certain non-tissue piercing electrodes **722** selected for atrial pacing and/or atrial sensing may be selected based on atrial capture threshold tests, electrode impedance, P-wave signal strength in the cardiac electrical signal, or other factors. For example, a single one or any combination of two or more individual non-tissue piercing electrodes **722** functioning as a cathode electrode that provides an optimal combination of a low pacing capture threshold amplitude and relatively high electrode impedance may be selected to achieve reliable atrial pacing using minimal current drain from the power source **98.**

In some instances, the distal-facing surface **738** may uniformly contact the atrial endocardial surface when the tissue piercing electrode **712** anchors the housing **730** at the implant site. In that case, all the electrodes **722** may be selected together to form the atrial cathode. Alternatively, every other one of the electrodes **722** may be selected together to form a multi-point atrial cathode having a higher electrical impedance that is still uniformly distributed along the distal-facing surface **738.** Alternatively, a subset of one or more electrodes **722** along one side of the insulative distal member **772** may be selected to provide pacing at a desired site that achieves the lowest pacing capture threshold due to the relative location of the electrodes **722** to the atrial tissue being paced.

In other instances, the distal-facing surface **738** may be oriented at an angle relative to the adjacent endocardial surface depending on the positioning and orientation at which the tissue piercing electrode **712** enters the cardiac tissue. In this situation, one or more of the non-tissue piercing electrodes **722** may be positioned in closer contact with the adjacent endocardial tissue than other non-tissue piercing electrodes **722,** which may be angled away from the endocardial surface. By providing multiple non-tissue piercing electrodes along the periphery of the insulative distal member **772,** the angle of the tissue piercing electrode **712** and the housing distal end region **732** relative to the cardiac surface, e.g., the right atrial endocardial surface, may not be required to be substantially parallel. Anatomical and positional differences may cause the distal-facing surface **738** to be angled or oblique to the endocardial surface, however, multiple non-tissue piercing electrodes **722** distributed along the periphery of the insulative distal member **772** increase the likelihood of good contact between one or more electrodes **722** and the adjacent cardiac tissue to promote acceptable pacing thresholds and reliable cardiac event sensing using at least a subset of multiple electrodes **722.** Contact or fixation circumferentially along the entire periphery of the insulative distal member **772** may not be required.

The non-tissue piercing electrodes **722** are shown to each include a first portion **722a** extending along the distal-facing surface **738** and a second portion **722b** extending along the circumferential surface **739.** The first portion **722a** and the second portion **722b** may be continuous exposed surfaces such that the active electrode surface wraps around a peripheral edge **776** of the insulative distal member **772** that joins the distal facing surface **738** and the circumferential surface **739.** The non-tissue piercing electrodes **722** may include one or more of the electrodes **772** along the distal-facing surface **738,** one or more electrodes along the circumferential surface **739,** one or more electrodes each extending along both of the distal-facing surface **738** and the circumferential surface **739,** or any combination thereof. The exposed surface of each of the non-tissue piercing electrodes **722** may be flush with respective distal-facing surfaces **738** and/or circumferential surfaces. In other examples, each of the non-tissue piercing electrodes **722** may have a raised surface that protrudes from the insulative distal member **772.** Any raised surface of the electrodes **722,** however, may define a smooth or rounded, non-tissue piercing surface.

The distal fixation and electrode assembly **736** may seal the distal end region of the housing **730** and may provide a foundation on which the electrodes **722** are mounted. The electrodes **722** may be referred to as housing-based electrodes. The electrodes **722** may not be not carried by a shaft or other extension that extends the active electrode portion away from the housing **730,** like the distal tip electrode **742** residing at the distal tip of the helical shaft **740** extending away from the housing **730.** Other examples of non-tissue piercing electrodes presented herein that are coupled to a distal-facing surface and/or a circumferential surface of an insulative distal member include the distal housing-based ring electrode **22** (**FIG. 3**), the distal housing-based ring electrode extending circumferentially around the assembly **36** (**FIG. 3**), button electrodes, other housing-based electrodes, and other circumferential ring electrodes. Any non-tissue piercing electrodes directly coupled to a distal insulative member, peripherally to a central tissue-piercing electrode, may be provided to function individually, collectively, or in any combination as a cathode electrode for delivering pacing pulses to adjacent cardiac tissue. When a ring electrode, such as the distal ring electrode **22** and/or a circumferential ring electrode, is provided, portions of the ring electrode may be electrically insulated by a coating to provide multiple distributed non-tissue piercing electrodes along the distal-facing surface and/or the circumferential surface of the insulative distal member.

The non-tissue piercing electrodes **722** and other examples listed above are expected to provide more reliable and effective atrial pacing and sensing than a tissue piercing electrode provided along the distal fixation and electrode assembly **736.** The atrial chamber walls are relatively thin compared to ventricular chamber walls. A tissue piercing atrial cathode electrode may extend too deep within the atrial tissue leading to inadvertent sustained or intermittent capture of ventricular tissue. A tissue piercing atrial cathode electrode may lead to interference with sensing atrial signals due to ventricular signals having a larger signal strength in the cardiac electrical signal received via tissue-piercing atrial cathode electrodes that are in closer physical proximity to the ventricular tissue. The tissue piercing electrode **712** may be securely anchored into ventricular tissue for stabilizing the implant position of the device **710** and providing reasonable certainty that the tip electrode **742** is sensing and pacing in ventricular tissue while the non-tissue piercing electrodes **722** are reliably pacing and sensing in the atrium. When the device **710** is implanted in the target implant region **4,** e.g., as shown in **FIG. 1** the ventricular septum, the tip electrode **742** may reach left ventricular tissue for pacing of the left ventricle while the non-tissue piercing electrodes **722** provide pacing and sensing in the right atrium. The tissue piercing electrode **712** may be in the range of about 4 to about 8 mm in length from the distal-facing surface **738** to reach left ventricular tissue. In some instances, the device **710** may achieve four-chamber pacing by delivering atrial pacing pulses from the atrial pacing circuit **83** via the non-tissue piercing electrodes **722** in the target implant region **4** to achieve bi-atrial (right and left atrial) capture and by delivering ventricular pacing pulses from the ventricular pacing circuit **85** via the tip electrode **742** advanced into ventricular tissue from the target implant region **4** to achieve biventricular (right and left ventricular) capture.

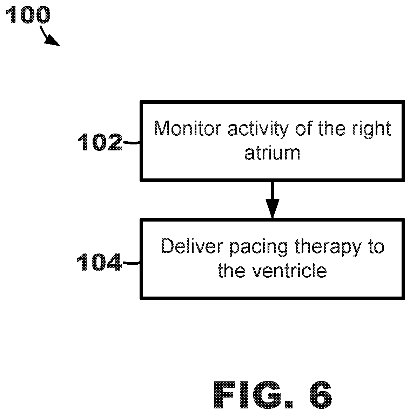

**FIGS. 6-14** relate to various methods that may be used to position a tissue-piercing electrode of any of the devices described herein, for example, those shown in **FIGS. 1-5** and **16-18.** In particular, each of the methods may be used when the tissue-piercing electrode is positioned, or implanted, in the basal and/or septal region of the left ventricular myocardium of the patient's heart from the triangle of Koch region of the right atrium through the right atrial endocardium and central fibrous body.

**FIG. 6** is a flowchart of a cardiac therapy method **100** for use with, e.g., the system **2** of **FIG. 1****.** For example, the method **100** may be used with the intracardiac medical device **10** (**FIGS. 1-2**) or **710** (**FIG. 5**). The method **100** may be used to describe single chamber or multiple chamber pacing (e.g., dual or triple chamber pacing), atrioventricular synchronous pacing, triggered pacing, cardiac resynchronization pacing, or tachycardia-related therapy.

The method **100** may include process **102,** in which activity of the right atrium is monitored. The medical device may use one or more of a plurality of electrodes to monitor the electrical activity of the patient's heart. For example, when implanted, a right atrial electrode (e.g., proximal housing-based electrode **24** or distal housing-based electrode **22** of **FIG. 2**) may be positioned to sense electrical activity of the right atrium. In some embodiments, the right atrial electrode may be positioned in the volume of the right atrium or against the right atrial endocardial surface of the patient's heart. In some embodiments, the right atrial electrode may be used to deliver cardiac therapy to the right atrium of the patient's heart. The right atrial electrode may be leadlessly coupled to a housing of the medical device. For example, the right atrial electrode may be positioned at the distal end region of the housing or at a location proximal to the distal end region (e.g., the proximal end region of the housing).

In addition, or as an alternative, to using the right atrial electrode, the medical device may use a motion detector to monitor the mechanical activity of the patient's heart. For example, when implanted, a right atrial motion detector may be positioned to sense mechanical activity of the right atrium. In some embodiments, the right atrial motion detector may be positioned within the implantable medical device **10.** In some embodiments, the right atrial motion detector may be wirelessly coupled, or coupled using a lead, to the implantable medical device **10.** The motion detector may be coupled to the same or a different sensing circuit as the electrodes.

The method **100** may further include process **104,** in which pacing therapy is delivered to the ventricle. For example, in process **104,** pacing therapy may be delivered to the left ventricle, right ventricle, ventricular septum (e.g., higher posterior basal septum region), or other parts of the ventricular myocardium, in response to the monitored activity of the right atrium in process **102.** The monitored activity may include electrical activity, mechanical activity, or both electrical and mechanical activity (e.g., using a right atrial electrode and/or a right atrial motion detector). The medical device may use one or more of the plurality of electrodes to deliver cardiac therapy to the patient's heart. For example, when implanted, a tissue-piercing electrode (e.g., tip electrode **42**) may be positioned in the ventricular septum to deliver cardiac therapy to the left ventricle of the patient's heart. The tissue-piercing electrode may be leadlessly coupled to the housing. In some embodiments, the tissue-piercing electrode may be used to sense electrical activity of the left ventricle. The tissue-piercing electrode may extend from a distal end region of the housing.

As mentioned above, the method **100** may be used with cardiac resynchronization pacing (e.g., cardiac resynchronization therapy). In some embodiments using cardiac resynchronization pacing, a pace may be delivered to one or both ventricles in response to a sensed atrial or ventricular event. Examples of cardiac resynchronization pacing include biventricular pacing and monoventricular pacing. Monoventricular pacing may include left ventricular pacing, right ventricular pacing, or fusion pacing. Various methods of performing cardiac resynchronization pacing are described in U.S. Patent Application Pub. No. 2017/0340885 (Sambelashvili), filed 31 July 2017, entitled "Systems and methods for leadless cardiac resynchronization therapy," and U.S. Patent No. 9,789,319 (Sambelashvili), issued 17 October 2017, entitled "Systems and methods for leadless cardiac resynchronization therapy," each of which is incorporated herein by reference in its entirety.

One type of cardiac resynchronization pacing is biventricular pacing. Biventricular pacing may include pacing the right ventricle (RV) with a RV electrode and a left ventricle (LV) with a LV electrode (e.g., the tissue-piercing electrode), which are typically different electrodes. In one or more embodiments, the implantable medical device can be configured to automatically switch between biventricular pacing and fusion pacing. In general, the primary goal may be to ensure the ventricles are synchronized with each other. Monoventricular pacing, or specifically fusion pacing, may be used instead of biventricular pacing to achieve synchrony. Skilled artisans appreciate that a patient's heart may be treated using adaptive CRT in which biventricular pacing may be delivered during one period of time (e.g., 1 hour, day, week etc.) and at another time, fusion pacing may be delivered to return the ventricles to synchrony. Typically, fusion pacing involves pacing the LV. However, there may be conditions for which the RV may be solely paced.

One type of adaptive CRT is adaptive LV pacing. Adaptive LV pacing may be described as leveraging intrinsic RV conduction by pre-pacing the LV to synchronize with intrinsic RV activation. The timing of the LV pace may be automatically adjusted based on the atrial to intrinsic QRS interval measurement (AV interval). One or more embodiments can set the LV pace to occur at about 70% of the intrinsic AV interval, but at least 40 ms prior to the intrinsic QRS.

One or more other embodiments can configure LV pacing in response to, or based on, a moderately lengthened QRS. For example, if the QRS width exceeds 120 ms, but does not exceed 160 ms, then LV pacing with fusion is selected. Otherwise, if the QRS width is greater than 160 ms, then biventricular (BiV) pacing is selected. Implementing a moderately lengthened QRS threshold may benefit heart failure patients. Efficacies of LV-only pacing or biventricular pacing may be predicted by the moderately lengthened QRS duration. An illustrative moderately lengthened QRS may correspond to a QRS width in the range of 130 ms-150 ms.

Adaptive CRT may use intrinsic AV conduction to determine whether to use biventricular or fusion pacing. In one or more embodiments, the intrinsic AV conduction may be automatically evaluated. In one or more embodiments, the IMD (e.g., an ICD), leadless pacing device (LPD) (e.g., an intracardiac medical device), and/or subcutaneous implantable device (SD) may automatically evaluate intrinsic ventricular conduction based upon QRS duration from the far-field electromyograph (EGM), or right ventricular sense to left ventricular sense (RVs-LVs) interval from the IMD sensing markers is automatically evaluated by the IMD or SD. For further detail, U.S. Pat. No. 4,374,382 issued to Markowitz et al. describes IMD sensing markers, which is incorporated herein by reference in its entirety. Based on the results, fusion pacing (i.e., LV only pacing or RV only pacing) or biventricular pacing may be selected. For example, RVs-LVs interval not exceeding 150 ms could correspond to LV only pacing, whereas >150 ms could switch the algorithm to biventricular pacing. In one or more other embodiments, RVs-LVs interval not exceeding 80 ms corresponds to fusion pacing while greater than 80 ms switches to biventricular pacing. Typically, RVs-LVs are shorter than the corresponding QRS width. Therefore, it takes about 40 ms to sense the onset of QRS in the RV and the final portion of the QRS in the LV is also sensed prior to the QRS end.

Various devices may be used to carry out the functionality of adaptive CRT. In one or more other embodiments, the IMD may track the moderately lengthened QRS over time and then relies on trend data to switch between biventricular pacing and fusion pacing. For example, assume that the moderately lengthened QRS is 120 ms, 125 ms, 130 ms, 135 m, 140 ms, and 145 ms, respectively for 6 consecutive weeks. The increasing trend could trigger the switch to biventricular pacing before the threshold is met for switching to biventricular pacing.

In another embodiment, the SD could send a control signal to the LPD to initiate CRT. The LPD could sense a cardiac signal (i.e., a second electrical signal) from the heart of the patient. Based on the cardiac signal, the LPD could determine whether to deliver CRT to the heart from the LPD. For example, the LPD, based on the second electrical signal, could determine that CRT is not necessary. The LPD could consider whether sensed data meets a pre-specified threshold. For instance, if the QRS width does not exceed 120 ms, the LPD may withhold the delivery of CRT therapy (e.g., the LPD could then signal the SD that CRT should not be delivered based upon the cardiac signal). The SD can be configured to perform a more detailed analysis in which at least one or more parameters (such as at least two parameters) are evaluated. The SD could then send another command signal that confirms, denies, or overrides the LPD.

In another embodiment, the LPD could sense a cardiac signal that indicates a switch between fusion pacing to biventricular pacing should occur and would signal the SD. The SD could be configured to send an override signal to the LPD unless certain conditions are met.

In yet another embodiment, the LPD could determine that biventricular pacing is may be used instead of fusion pacing in contravention to the SD communication. In one embodiment, the LPD would deliver biventricular pacing. In one or more other embodiments, the LPD could determine that fusion pacing may be used instead of biventricular pacing in contravention to the SD communication. In this scenario, the LPD could deliver fusion pacing.

In another embodiment, the SD transmits a control signal to the LPD to initiate CRT. The LPD senses a cardiac signal (i.e., a second electrical signal) from the heart of the patient. Based on the cardiac signal, the LPD could determine whether to deliver CRT or the type of CRT to deliver to the heart from the LPD. In one or more embodiments, the LPD, based on the second electrical signal, could initially determine that CRT should not be used. The initial determination by the LPD could use tests such as a threshold of one or more parameters. In one or more embodiments, the SD could perform a more detailed analysis as to whether CRT should be delivered. Using the sensed data from the LPD and/or SD, the SD could generate another signal to the LPD that either confirms, denies or overrides the LPDs initial determination.

In another embodiment, the LPD could sense a cardiac signal that indicates a switch should occur between fusion pacing to biventricular pacing. Determining whether to switch between fusion pacing and biventricular pacing could be determined based upon one or more parameters (e.g., moderately lengthened QRS, etc.). The LPD could be configured to either automatically switch between fusion pacing and biventricular pacing or to wait until the SD confirms or denies switching between the CRT pacing mode (i.e., fusion pacing and biventricular pacing). The SD could be configured to send a confirmatory signal or a signal denying the LPD switching the pacing mode.

In yet another embodiment, the LPD could determine that biventricular pacing may be used instead of fusion pacing in contravention to the SD communication. In one embodiment, the LPD would deliver biventricular pacing. In one or more other embodiments, the LPD could determine that fusion pacing is required over biventricular pacing in contravention to the SD communication. In this scenario, the LPD could deliver fusion pacing.

In one or more other embodiments, a device having SD-like functionality is implanted into a patient's heart. For example, the SD could function as a conventional ICD or have the SD functionality described herein. Electrical signals are then sensed which includes moderately lengthened QRS duration data from the patient's heart. A determination is made as to whether cardiac resynchronization pacing therapy (CRT pacing) is appropriate based upon the moderately lengthened QRS duration in the sensed electrical signals. The CRT pacing pulses are delivered to the heart using electrodes. In one or more embodiments, the SD can switch between fusion pacing and biventricular pacing based upon data (e.g., moderately lengthened QRS, etc.) sensed from the heart.

In addition, there are even further embodiments that may be implemented with the methods described herein. One or more LPDs carrying one or more electrodes may be implanted within various chambers of the heart of the patient or otherwise in close proximity of the cardiac muscle. At these locations, an LPD may sense ECG signals with high signal-to-noise ratios to detect arrhythmias. In addition, an LPD may provide cardiac pacing at the location of the implanted LPD. In some examples, one or both of SD and LPD may share detected signals or physiological information (e.g., R-R intervals, electrogram morphology measurements, and/or electrocardiograms or electrograms) such that the device receiving such information can determine a condition of patient (e.g., determine whether or not patient is experiencing an arrhythmia and or lack of synchrony between ventricles). Communication between an LPD and a subcutaneous ICD (SICD) is described in U.S. patent application Ser. No. 13/756,085, filed on Jan. 31, 2013, which is incorporated herein by reference in its entirety.

In some examples, communication between the SICD and an LPD may be used to initiate therapy and/or confirm that therapy should be delivered. The SICD may also transmit a communication message to the LPD instructing the LPD to change one or more parameters that define the CRT therapy. In this one-way communication example, the SICD may be configured to transmit communications to the LPD and the LPD may be configured to receive the communication from the SICD. Alternatively, one-way communication may be established such that the LPD may be configured to transmit communications to the SICD (e.g., communication from an LPD). In other examples, two-way communication may allow confirmation of a detected of a cardiac condition (e.g., ventricular dyssynchrony, tachyarrhythmia, bradycardia, etc.) prior to delivery of any therapy. Communication between the SD and the LPD is described in greater details in U.S. Patent Application Ser. No. 13/756,085 (Attorney Docket No. C0001726.USU1) filed May 26, 2013 and entitled "Systems and methods for leadless pacing and shock therapy," which is incorporated herein by reference in its entirety.

One or more of the embodiments described herein may utilize far-field sensing to carry out cardiac resynchronization pacing. In some embodiments, one or more of the electrodes of a device may be used for far-field sensing of electrical activity in a different chamber. For example, a right atrial electrode or a tissue-piercing electrode may be used to monitor far-field electrical activity of the right ventricle. The far-field sensing may be particularly useful in cardiac resynchronization pacing as described above. Ascertaining RV timing may be used to tailor the timing of the LV pace for an individual patient to allow for fusion pacing (e.g., fusing RV and LV activation).

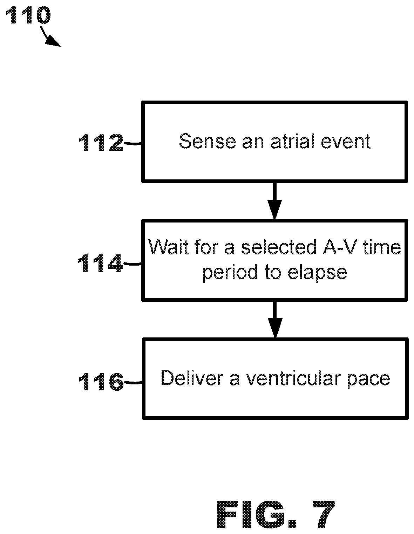

**FIG. 7** is a flowchart of a pacing therapy method **110** for use with, e.g., the method **100** of **FIG. 6****.** For example, the method **110** may be used in process **104,** which delivers pacing therapy to the ventricle. The method **110** may be described as an AV resynchronization therapy method.

The method **110** may include process **112,** in which an atrial event is sensed. For example, the atrial event may be a right atrial event in monitored activity of the right atrium, e.g., as mentioned in process **102.** The right atrial electrode may be used to sense the right atrial event within monitored electrical activity of the right atrium. In addition, or alternatively, the right atrial motion detector may be used to sense the right atrial event within monitored mechanical activity of the right atrium. The atrial event may be determined by the controller of the medical device using the sensing circuit.

The method **110** may further include process **114,** which includes waiting for a selected A-V time period to elapse in response to the detection of the atrial event in process **112.** For example, the controller of the medical device may wait the selected A-V time period after detecting the atrial event.

The method **110** may further include process **116,** in which a ventricular pace is delivered in response to the atrial event after the selected A-V time period has elapsed in process **114.** In some embodiments, process **116** may be performed without process **114.** It is contemplated that the ventricular pace may be delivered in response to sensing the atrial event without using the selected A-V time period.

In some embodiments, the A-V time period is configured to synchronize an atrial event and a ventricular event for atrioventricular synchrony. The A-V time period may also be configured to, or modified to, improve cardiac resynchronization. For example, the A-V time period may be used to pace one or both ventricles for ventricular synchrony (e.g., using biventricular pacing or monoventricular pacing, such as fusion pacing) described herein in more detail below.

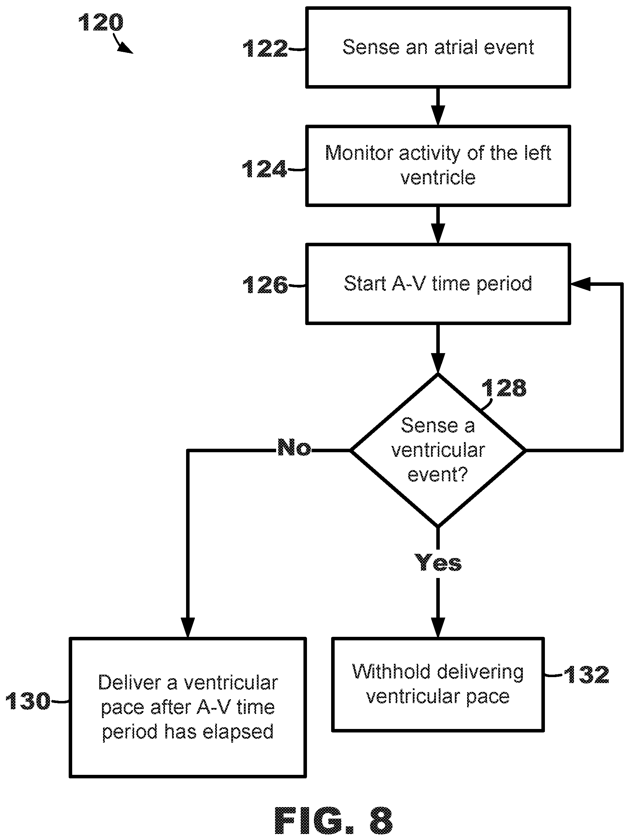

**FIG. 8** is a flowchart of another illustrative pacing therapy method **120** for use with, e.g., the method **100** of **FIG. 6****.** For example, the method **120** may be used in process **104,** which delivers pacing therapy to the ventricle. The method **120** may be described as an AV resynchronization therapy method.

The method **120** may include process **122,** in which an atrial event is sensed within the monitored activity from process **102** (**FIG. 6**). Process **122** may be the same or similar to process **112** of method **110** (**FIG. 7**).

The method **120** may further include process **124,** in which activity of the left ventricle is monitored. One or more tissue-piercing electrodes may be used to sense the activity of the left ventricle. If more than one tissue-piercing electrode has contact with LV myocardium, the tissue-piercing electrodes can be used to both sense and pace the LV.

The method **120** may further include process **126,** which starts an A-V time period. The method **120** may further include process **128,** which determines whether a ventricular event is sensed in response to the monitored activities in processes **122** and **124.** In particular, the process **128** may determine whether a ventricular event is sensed before the A-V time period elapses. The controller of the medical device may be used to make the determination. If a ventricular event is not sensed in process **128,** the method **120** may branch to process **130.** If a ventricular event is sensed, the method **120** may branch to process **132.**

The method **120** may further include process **130,** in which a ventricular pace is delivered after the selected A-V time period has elapsed. Process **130** may be the same or similar to process **114** of method **110** (**FIG. 7**). The controller of the medical device may initiate or execute the ventricular pace using the therapy delivery circuit.

The method **120** may further include process **132,** which withholds delivering the ventricular pace. Withholding the ventricular pace delivery may be described as inhibiting pacing therapy.

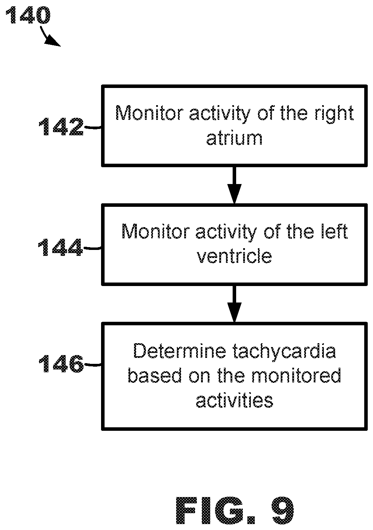

**FIG. 9** is a flowchart of a tachycardia-related method **140** for use with, e.g., the system **2** of **FIG. 1****.** For example, the method **140** may be used with the intracardiac medical device **10** (**FIGS. 1-2**) or **710** (**FIG. 5**). The method **140** may be described as a tachycardia sensing method.

The method **140** may include process **142,** in which activity of the right atrium is monitored. When implanted, the controller of the medical device may use the sensing circuit and right atrial electrode or right atrial motion detector to monitor the activity of the right atrium.

The method **140** may further include process **144,** in which activity of the left ventricle is monitored. When implanted, the controller of the medical device may use the sensing circuit and tissue-piercing electrode, or a left ventricular motion detector, to monitor the activity of the left ventricle.

The method **140** may further include process **146,** in which tachycardia is determined based on the monitored activities in processes **142** and **144.** The controller may be used to make the determination.

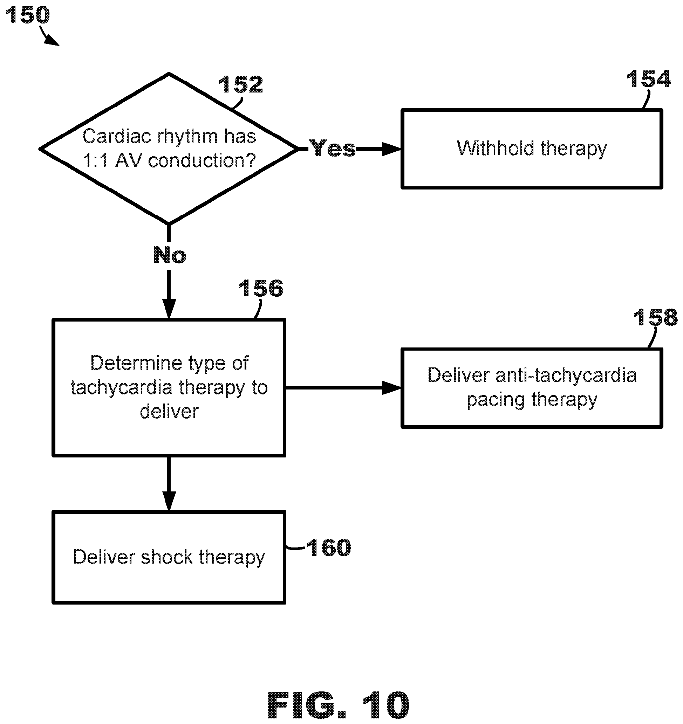

**FIG. 10** is a flowchart of a tachycardia determination method **150** for use with, e.g., the method **140** of **FIG. 9****.** For example, the method **150** may be used in process **146,** which determines tachycardia based on the monitored activities. The method **150** may be described as a tachycardia classification method. A ventricular tachycardia event may be determined if the monitored V-rate exceeds a certain threshold (e.g., 120 bpm) or, additionally or alternatively, if the monitored intervals between successive V-events are less than a certain time-interval threshold (e.g., 500 ms).

The method **150** may include process **152,** which determines whether a tachycardia rhythm has a 1:1 AV conduction rhythm or signature. As used herein, a 1:1 AV conduction or rhythm refers to every V-event being preceded by an A-event with a degree of regularity in terms of the A-V interval. The controller of the medical device may be used to make the determination. If a 1:1 AV conduction rhythm is determined, the method **150** may continue to process **154.** If a 1:1 AV conduction rhythm is not determined by process **152,** then the method **150** may continue to process **156.**

The method **150** may further include process **154,** in which therapy is withheld. For example, the controller may be used to withhold tachycardia-related therapy in response to determining that the detected rhythm is non-treatable.

The method **150** may further include process **156** (e.g., in response to not determining a 1:1 AV conduction rhythm in process **152**), in which a type of tachycardia therapy to deliver is determined. For example, a determination may be made that the V-rate is faster than a rate threshold (e.g., 200 bpm) for ventricular fibrillation, in which case the device may initiate shock therapy in process **160.** If not, then the device may initiate anti-tachycardia pacing therapy in process **158.**

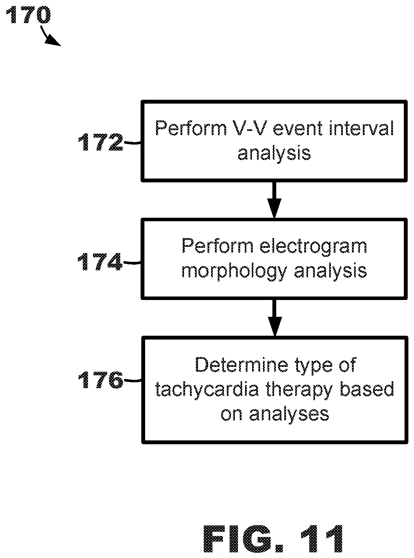

**FIG. 11** is a flowchart of a tachycardia therapy determination method **170** for use with, e.g., the method **150** of **FIG. 10****.** For example, the method **170** may be used in process **156,** which determines a type of tachycardia therapy to deliver.

The method **170** may include process **172,** which performs a V-V event interval analysis, which includes determination of parameters like median, range, mode-sum, or other metrics reflective of how fast a tachycardia rhythm is and how regular is the interval between successive V-events. The method **170** may further include process **174,** which performs electrogram morphology analysis. This may include comparing entire morphology or gross morphologic features of different V-events within a tachycardia rhythm and determining a morphologic similarity index. Details of determining morphologic and rate regularity of tachycardia events may be described in U.S. Patent No. 8,594,775 (Ghosh et al.), which is incorporated herein by reference in its entirety.

The method **170** may further include process **176,** in which rate and rhythm regularity of tachycardia events are determined based on combination of monitoring of intervals between successive V events (process **172**) and corresponding electrogram morphologies (process **174**). In some embodiments, a monomorphic VT may be determined based on regularity of the successive V-events and/or similarity of electrogram morphologies corresponding to the V-events, whereas a polymorphic rhythm may be determined if both rate regularity and morphology similarity criteria are not met.

Non-limiting examples of tachycardia therapy that may be delivered include anti-tachycardia pacing and shock therapy (e.g., defibrillation therapy). In some embodiments, if a monomorphic VT is determined, the device may initiate anti-tachycardia pacing (ATP). If a polymorphic rhythm is determined the device may initiate shock therapy by signaling the external device to deliver a shock.

Examples of tachycardia therapy determination methods, including interval analyses and electrogram morphology analyses, that may be used with the methods of the present disclosure may be described in U.S. Patent No. 7,031,711 (Brown et al.), issued 18 April 2016, and U.S. Patent No. 8,594,775 (Ghosh et al.), issued 26 November 2013, and U.S. Patent No. 8,750,994 (Ghosh et al.), issued 10 June 2014, each of which is incorporated herein by reference in its entirety.

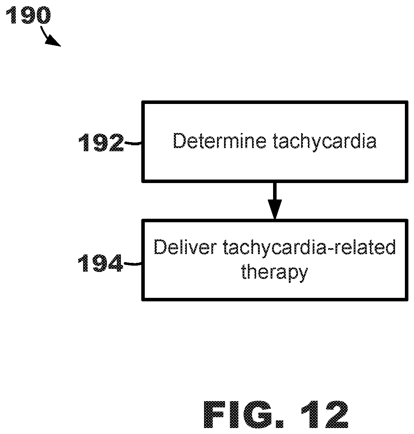

**FIG. 12** is a flowchart of an anti-tachycardia method **190** for use with, e.g., the system **2** of **FIG. 1****.** For example, the method **190** may be used with the intracardiac medical device **10** and the separate medical device **50** (**FIG. 1**).

The method **190** may include process **192,** which determines that tachycardia is present. For example, the controller of the medical device may determine that tachycardia is present based on monitored activities generated using the plurality of electrodes and/or one or more motion detectors. Process **192** may be the same or similar to process **146** of **FIG. 9** or process **176** of **FIG. 11****.**

The method **190** may further include process **194,** which delivers tachycardia-related therapy using the medical device and/or separate medical device based on the determination that tachycardia is present in process **192.** For example, the medical device may use one or more of the plurality of electrodes to provide anti-tachycardia pacing.

In some embodiments, another device may perform process **192** to determine whether tachycardia has been detected and the medical device may be used to perform process **194** to provide the tachycardia-related therapy.

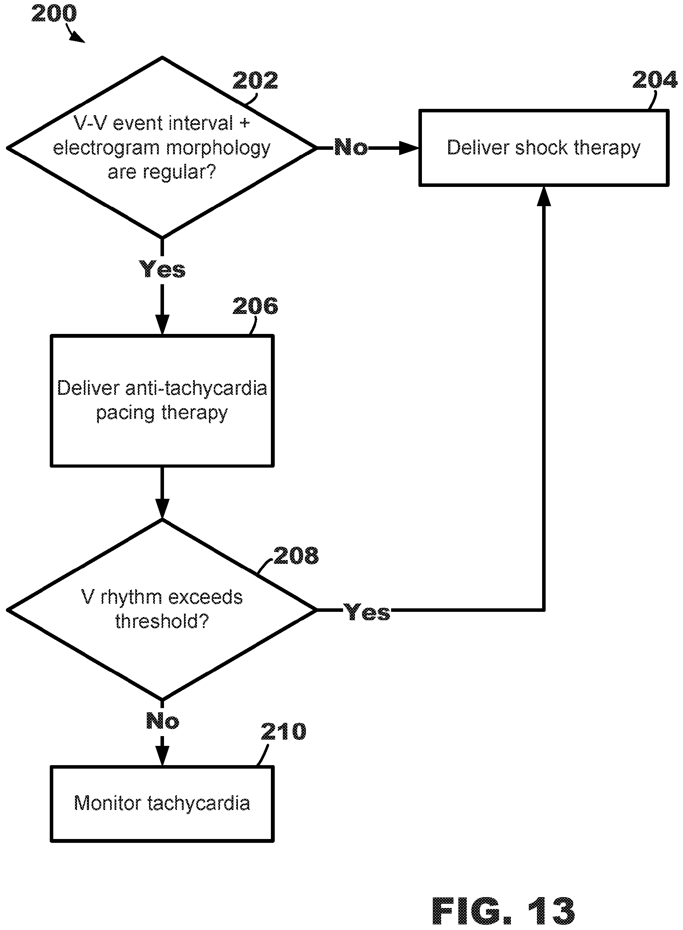

**FIG. 13** is a flowchart of a tachycardia therapy delivery method **200** for use with, e.g., the method **190** of **FIG. 12****.** For example, the method **200** may be included in process **194** of method **190,** which delivers tachycardia-related therapy.

The method **200** may include process **202,** which may be similar to process **176** of **FIG. 11** that determines a type of tachycardia to deliver. In particular, process **202** may determine whether the V-V event interval (determined in process **172** of method **170**) and the electrogram morphology (determined in process **174** of method **170**) are both regular. If one or both are irregular, the method **200** may continue with process **204.** If both are regular, the method **200** may continue with process **206.**

The method **200** may further include process **204** to deliver shock therapy. Shock therapy may be initiated using the medical device. For example, the medical device may deliver a signaling pulse (e.g., a distinctive single, high-output pacing pulse to the ventricle) or other trigger. The pacing signal or other trigger may be detected by the separate medical device, which may provide the electrical shock.

The method **200** may further include process **206** to deliver anti-tachycardia pacing therapy (ATP). The ATP may be delivered using the plurality of electrodes of the medical device. After process **206,** the method **200** may continue with process **208.**

The method **200** may include process **208** to determine whether the V rhythm exceeds a threshold after ATP delivery has begun in process **206.** In particular, a V rhythm threshold may be used to distinguish monomorphic VT from other types of tachycardia, in the same or similar manner as process **156** of method **150** (**FIG. 10**). If the V rhythm exceeds the V rhythm threshold, the method **200** may continue to process **204** described above. If the V rhythm does not exceed the V rhythm threshold, the method may continue with process **210.**

The method may further include process **210** to monitor tachycardia. For example, process **210** may include performing the method **170** of **FIG. 11****.** After process **210,** the method **200** may return to process **202.** While the V-V event interval and the electrogram morphology are regular, the method **200** may continue to loop, for example, unless shock therapy is delivered in process **204.**

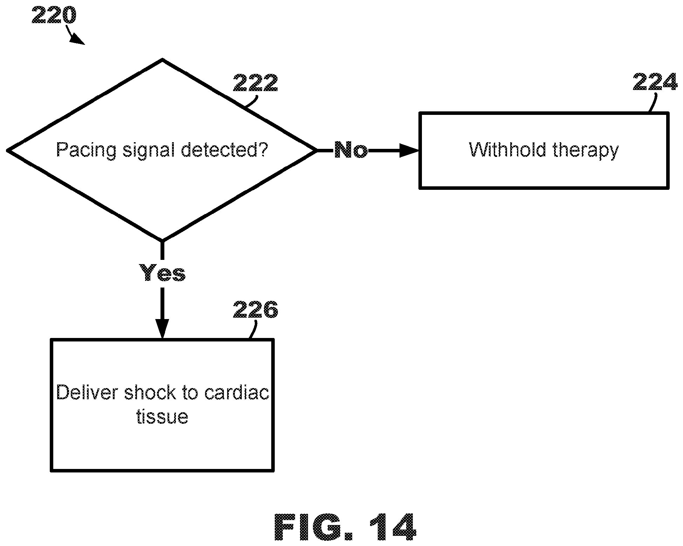

**FIG. 14** is a flowchart of a shock therapy method **220** for use with, e.g., the method **200** of **FIG. 13****.** For example, the method **220** may be included in process **204,** which delivers shock therapy. The method **220** may be executed by the separate medical device **50** of **FIG. 1****.**

The method **220** may begin with process **222,** which determines whether a pacing signal has been detected. As described with respect to process **204** of **FIG. 13****,** the medical device may provide a pacing signal, which may be detected by the separate medical device. If the pacing signal is not detected, the method **220** may continue to process **224.** If the pacing signal is detected, the method **220** may continue with process **226.**

The method **220** may further include process **224** to withhold therapy. In particular, process **224** may withhold shock therapy from being delivered by the separate medical device (e.g., extravascular ICD).

The method **220** may further include process **226** to deliver a shock to cardiac tissue of the patient's heart. In particular, the separate medical device may be used to provide the shock as, e.g., the medical device may not have a sufficient battery capacity, size, or positioning sufficient to deliver an effective cardioversion or defibrillation shock.

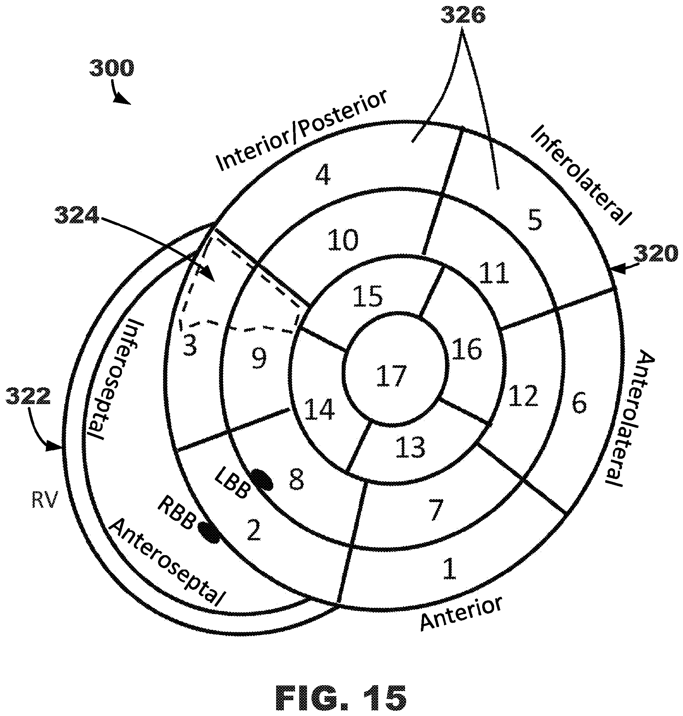

**FIG. 15** is a two-dimensional (2D) ventricular map **300** of a patient's heart (e.g., a top-down view) showing the left ventricle **320** in a standard 17 segment view and the right ventricle **322.** The map **300** includes a plurality of areas **326** corresponding to different regions of a human heart. As illustrated, the areas **326** are numerically labeled 1-17 (which, e.g., correspond to a standard 17 segment model of a human heart, correspond to 17 segments of the left ventricle of a human heart, etc.). Areas **326** of the map **300** may include basal anterior area 1, basal anteroseptal area 2, basal inferoseptal area 3, basal inferior area 4, basal inferolateral area 5, basal anterolateral area 6, mid-anterior area 7, mid-anteroseptal area 8, mid-inferoseptal area 9, mid-inferior area 10, mid-inferolateral area 11, mid-anterolateral area 12, apical anterior area 13, apical septal area 14, apical inferior area 15, apical lateral area 16, and apex area 17. The inferoseptal and anteroseptal areas of the right ventricle **322** are also illustrated, as well as the right bunch branch (RBB) and left bundle branch (LBB).

In some embodiments, any of the tissue-piercing electrodes of the present disclosure may be implanted in the basal and/or septal region of the left ventricular myocardium of the patient's heart. In particular, the tissue-piercing electrode may be implanted from the triangle of Koch region of the right atrium through the right atrial endocardium and central fibrous body.

Once implanted, the tissue-piercing electrode may be positioned in the target implant region **4** (**FIG. 1**), such as the basal and/or septal region of the left ventricular myocardium. With reference to map **300,** the basal region includes one or more of the basal anterior area 1, basal anteroseptal area 2, basal inferoseptal area 3, basal inferior area 4, mid-anterior area 7, mid-anteroseptal area 8, mid-inferoseptal area 9, and mid-inferior area 10. With reference to map **300,** the septal region includes one or more of the basal anteroseptal area 2, basal anteroseptal area 3, mid-anteroseptal area 8, mid-inferoseptal area 9, and apical septal area 14.

In some embodiments, the tissue-piercing electrode may be positioned in the basal septal region of the left ventricular myocardium when implanted. The basal septal region may include one or more of the basal anteroseptal area 2, basal inferoseptal area 3, mid-anteroseptal area 8, and mid-inferoseptal area 9.

In some embodiments, the tissue-piercing electrode may be positioned in the high inferior/posterior basal septal region of the left ventricular myocardium when implanted. The high inferior/posterior basal septal region of the left ventricular myocardium may include a portion of at least one of the basal inferoseptal area 3 and mid-inferoseptal area 9. For example, the high inferior/posterior basal septal region may include region **324** illustrated generally as a dashed-line boundary. As shown, the dashed line boundary represents an approximation of about where the high inferior/posterior basal septal region and may take somewhat different shape or size depending on the particular application. Without being bound by any particular theory, intraventricular synchronous pacing and/or activation may result from stimulating the high septal ventricular myocardium due to functional electrical coupling between the subendocardial Purkinje fibers and the ventricular myocardium.

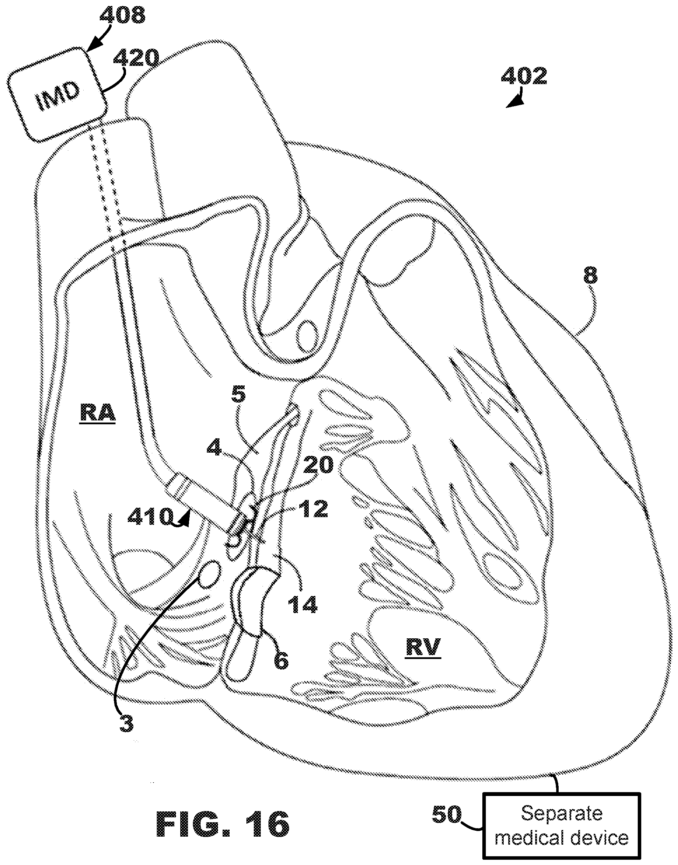

**FIGS. 16-18** are conceptual diagrams of illustrative cardiac therapy systems **402, 404, 406** including leaded medical devices **408, 418, 428** with electrodes implanted in a patient's heart **8.** Many of the structures of cardiac therapy systems **402, 404, 406** may be the same as those in cardiac therapy system **2.** Accordingly, many of the structures depicted in **FIGS. 16-18** have the same numbering as the structures depicted in **FIG. 1****.** The description of some reference characters shown in **FIGS. 16-18** (e.g., separate medical device **50**) may be found in the description above, in particular, with respect to **FIG. 1****,** and is not repeated here with reference to **FIGS. 16-18****.**

With reference to **FIG. 16****,** leaded medical device **408** includes one, or a single, implantable lead **410** having a tissue-piercing electrode **12** coupled to a distal end region of the lead and implanted inside the patient's heart **8.** The housing **420** of the leaded medical device **408** may be implanted and positioned outside of the patient's heart **8.** The lead **410** may include a right atrial electrode, and the device **408** may operate as a dual-channel capable device. In some embodiments, the lead **410** may not include a right atrial electrode. In other words, the leaded medical device **408** may be a single channel device, which may be used for asynchronous, triggered, or other single channel pacing. For example, lead **410** may sense activity or deliver pacing to the left ventricle (LV) when the tissue-piercing electrode **12** is implanted as described above (e.g., with respect to map **300** of **FIG. 15** and with respect to device **10** of **FIG. 1**).

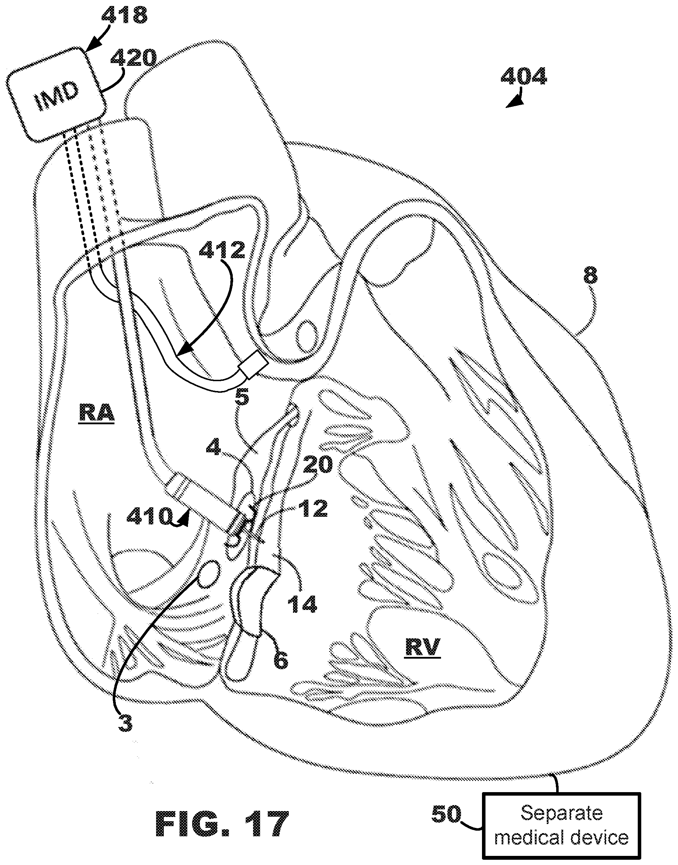

With reference to **FIG. 17****,** leaded medical device **418** is like leaded medical device **408** except that device **418** includes two implantable leads **410, 412.** In particular, implantable lead **412** may include an electrode (e.g., a right atrial electrode) coupled to a distal end region of the lead and may be implanted in a different location than lead **410.** In some embodiments, lead **412** is implanted in a different region of the right atrium. In some embodiments, each lead **410, 412** may contribute one channel of a dual-channel device **418.** For example, lead **410** may sense activity or deliver pacing to the left ventricle (LV), and lead **412** may sense activity or deliver pacing to the right atrium (RA).

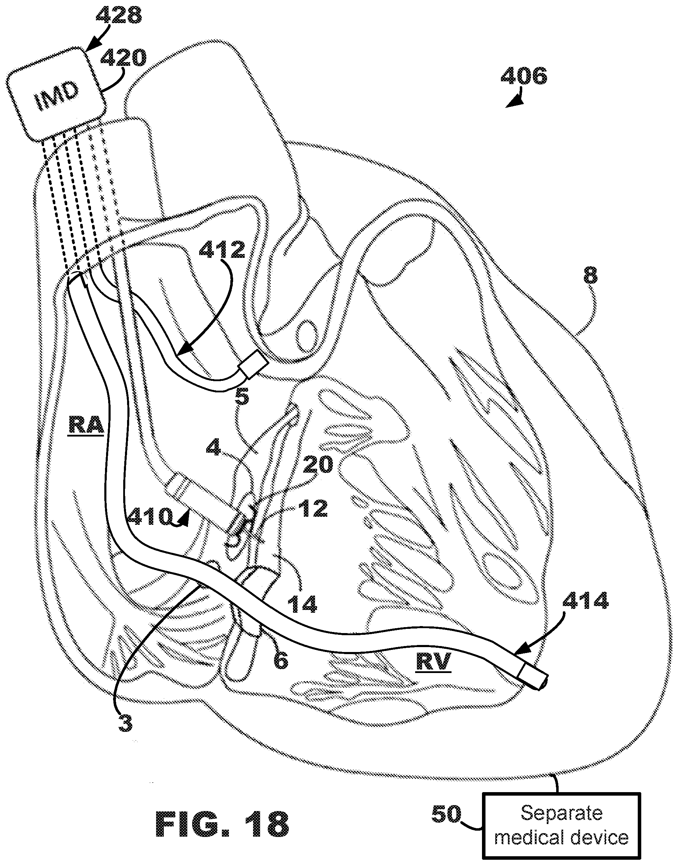

With reference to **FIG. 18****,** leaded medical device **428** is like leaded medical device **418** except that device **428** includes three implantable leads **410, 412, 414.** In particular, implantable lead **414** may include an electrode (e.g., a right ventricular electrode) coupled to a distal end region of the lead and may be implanted in a different location than leads **410, 412.** In some embodiments, lead **414** is implanted in a region of the right ventricle. In some embodiments, each lead **410, 412, 414** may contribute one channel to a multi-channel device **428.** For example, lead **410** may sense activity or deliver pacing to the left ventricle (LV), lead **412** may sense activity of deliver pacing to the right atrium (RA), and lead **414** may sense activity or deliver pacing to the right ventricle (RV).

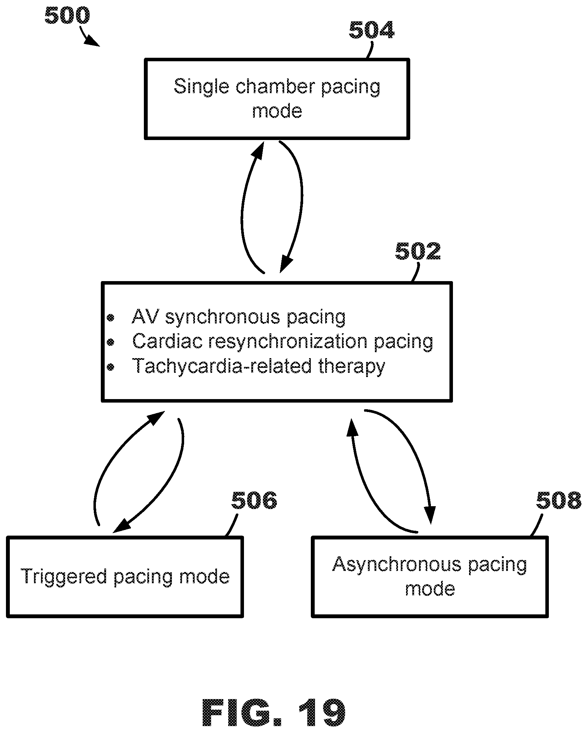

**FIG. 19** is a state diagram **500** showing different illustrative therapy modes that the implantable medical device and systems described herein may initiate or enter during operation. Such implantable medical devices and systems may have a nominal state **502,** in which the device is configured to perform at least one or more of atrioventricular synchronous pacing, cardiac resynchronization pacing, and tachycardia-related therapy. The device may initiate or switch to a single chamber pacing mode in state **504** in response to detecting atrial fibrillation. Atrial fibrillation may be detected by the device or by a separate device. The device may return to the nominal state **502** from state **504.**

The device may initiate or switch to a triggered pacing mode in state **506,** in which the device delivers pacing in response to a trigger (e.g., pacing signal) from a separate device (e.g., an ICD). In some embodiments, the implantable medical device may include only electrodes to pace the LV (e.g., tissue-piercing electrode), which is triggered by a separate, or remote, device (e.g., a subcutaneous or leaded device) that determines timing and sends a command or trigger to the implantable medical device to pace the LV to deliver CRT. The device may return to the nominal state **502** from state **506.**

Further, the device may initiate or switch to an asynchronous pacing mode in state **508,** in which the device delivers pacing independent of sensing in other chambers. The device may return to the nominal state **502** from state **508.**

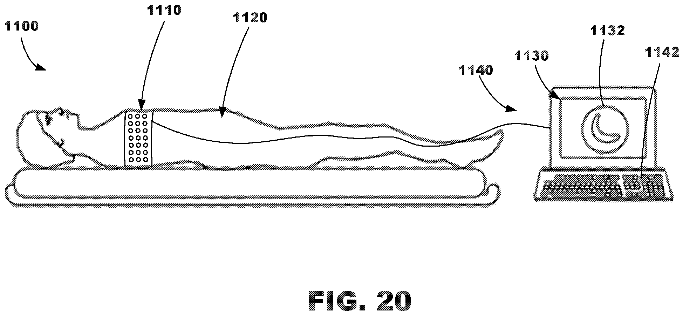

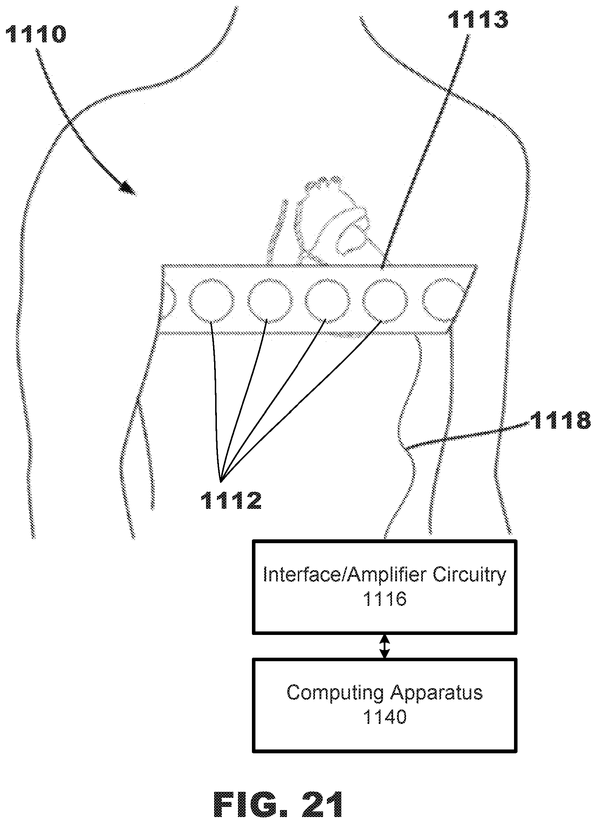

Reference will now be made to **FIGS. 20-22****,** which are diagrams of illustrative systems include electrode apparatus. An illustrative system **1100** including electrode apparatus **1110,** display apparatus **1130,** and computing apparatus **1140** is depicted in **FIG. 20****.** The electrode apparatus **1110** as shown includes a plurality of electrodes incorporated, or included, within a band wrapped around the chest, or torso, of a patient **1120.** The electrode apparatus **1110** is operatively coupled to the computing apparatus **1140** (e.g., through one or wired electrical connections, wirelessly, etc.) to provide electrical signals from each of the electrodes to the computing apparatus **1140** for analysis, evaluation, etc. Illustrative electrode apparatus may be described in U.S. Patent No. 9,320,446 entitled "Bioelectric Sensor Device and Methods" and issued on April 26, 2016, which is incorporated herein by reference in its entirety. Further, illustrative electrode apparatus **1110** will be described in more detail in reference to **FIGS. 21-22****.**

Although not described herein, the illustrative system **1100** may further include imaging apparatus. The imaging apparatus may be any type of imaging apparatus configured to image, or provide images of, at least a portion of the patient in a noninvasive manner. For example, the imaging apparatus may not use any components or parts that may be located within the patient to provide images of the patient except noninvasive tools such as contrast solution. It is to be understood that the illustrative systems, methods, and interfaces described herein may further use imaging apparatus to provide noninvasive assistance to a user (e.g., a physician) to locate and position a device to deliver VfA cardiac pacing therapy and/or to locate or select a pacing electrode or pacing vector proximate the patient's heart for Ventricle from atrium pacing therapy in conjunction with the evaluation of Ventricle from atrium pacing therapy.

For example, the illustrative systems, methods, and interfaces may provide image guided navigation that may be used to navigate leads including leadless devices, electrodes, leadless electrodes, wireless electrodes, catheters, etc., within the patient's body while also providing noninvasive cardiac therapy evaluation including determining whether a ventricle from atrium (VfA) paced setting is acceptable or determining whether one or more selected parameters are acceptable, such as selected location information (e.g., location information for the electrodes to target a particular location in the left ventricle). Illustrative systems and methods that use imaging apparatus and/or electrode apparatus may be described in U.S. Patent Publication No. 2014/0371832 filed on June 12, 2013 and entitled "Implantable Electrode Location Selection," U.S. Patent Publication No. 2014/0371833 filed on June 12, 2013 and entitled "Implantable Electrode Location Selection," U.S. Patent Publication No. 2014/0323892 filed on March 27, 2014 and entitled "Systems, Methods, and Interfaces for Identifying Effective Electrodes," U.S. Patent Publication No. 2014/0323882 filed on March 27, 2014 and entitled "Systems, Methods, and Interfaces for Identifying Optical Electrical Vectors," each of which is incorporated herein by reference in its entirety.

Illustrative imaging apparatus may be configured to capture x-ray images and/or any other alternative imaging modality. For example, the imaging apparatus may be configured to capture images, or image data, using isocentric fluoroscopy, bi-plane fluoroscopy, ultrasound, computed tomography (CT), multi-slice computed tomography (MSCT), magnetic resonance imaging (MRI), high frequency ultrasound (HIFU), optical coherence tomography (OCT), intra-vascular ultrasound (IVUS), two dimensional (2D) ultrasound, three dimensional (3D) ultrasound, four dimensional (4D) ultrasound, intraoperative CT, intraoperative MRI, etc. Further, it is to be understood that the imaging apparatus may be configured to capture a plurality of consecutive images (e.g., continuously) to provide video frame data. In other words, a plurality of images taken over time using the imaging apparatus may provide video frame, or motion picture, data. Additionally, the images may also be obtained and displayed in two, three, or four dimensions. In more advanced forms, four-dimensional surface rendering of the heart or other regions of the body may also be achieved by incorporating heart data or other soft tissue data from a map or from pre-operative image data captured by MRI, CT, or echocardiography modalities. Image datasets from hybrid modalities, such as positron emission tomography (PET) combined with CT, or single photon emission computer tomography (SPECT) combined with CT, could also provide functional image data superimposed onto anatomical data, e.g., to be used to navigate treatment apparatus proximate target locations (e.g., such as locations within the left ventricle, including a selected location within the high posterior basal septal area of the left ventricular cavity) within the heart or other areas of interest.

Systems and/or imaging apparatus that may be used in conjunction with the illustrative systems and method described herein are described in U.S. Pat. App. Pub. No. 2005/0008210 to Evron et al. published on January 13, 2005, U.S. Pat. App. Pub. No. 2006/0074285 to Zarkh et al. published on April 6, 2006, U.S. Pat. App. Pub. No. 2011/0112398 to Zarkh et al. published on May 12, 2011, U.S. Pat. App. Pub. No. 2013/0116739 to Brada et al. published on May 9, 2013, U.S. Pat. No. 6,980,675 to Evron et al. issued on December 27, 2005, U.S. Pat. No. 7,286,866 to Okerlund et al. issued on October 23, 2007, U.S. Pat. No. 7,308,297 to Reddy et al. issued on December 11, 2011, U.S. Pat. No. 7,308,299 to Burrell et al. issued on December 11, 2011, U.S. Pat. No. 7,321,677 to Evron et al. issued on January 22, 2008, U.S. Pat. No. 7,346,381 to Okerlund et al. issued on March 18, 2008, U.S. Pat. No. 7,454,248 to Burrell et al. issued on November 18, 2008, U.S. Pat. No. 7,499,743 to Vass et al. issued on March 3, 2009, U.S. Pat. No. 7,565,190 to Okerlund et al. issued on July 21, 2009, U.S. Pat. No. 7,587,074 to Zarkh et al. issued on September 8, 2009, U.S. Pat. No. 7,599,730 to Hunter et al. issued on October 6, 2009, U.S. Pat. No. 7,613,500 to Vass et al. issued on November 3, 2009, U.S. Pat. No. 7,742,629 to Zarkh et al. issued on June 22, 2010, U.S. Pat. No. 7,747,047 to Okerlund et al. issued on June 29, 2010, U.S. Pat. No. 7,778,685 to Evron et al. issued on August 17, 2010, U.S. Pat. No. 7,778,686 to Vass et al. issued on August 17, 2010, U.S. Pat. No. 7,813,785 to Okerlund et al. issued on October 12, 2010, U.S. Pat. No. 7,996,063 to Vass et al. issued on August 9, 2011, U.S. Pat. No. 8,060,185 to Hunter et al. issued on November 15, 2011, and U.S. Pat. No. 8,401,616 to Verard et al. issued on March 19, 2013, each of which is incorporated herein by reference in its entirety.

The display apparatus **1130** and the computing apparatus **1140** may be configured to display and analyze data such as, e.g., electrical signals (e.g., electrocardiogram data), cardiac information representative of at least one of mechanical cardiac functionality and electrical cardiac functionality, etc. Cardiac information may include, e.g., electrical heterogeneity information or electrical dyssynchrony information, surrogate electrical activation information or data, etc. that is generated using electrical signals gathered, monitored, or collected, using the electrode apparatus **1110.** In at least one embodiment, the computing apparatus **1140** may be a server, a personal computer, or a tablet computer. The computing apparatus **1140** may be configured to receive input from input apparatus **1142** and transmit output to the display apparatus **1130.** Further, the computing apparatus **1140** may include data storage that may allow for access to processing programs or routines and/or one or more other types of data, e.g., for driving a graphical user interface configured to noninvasively assist a user in targeting placement of a pacing device and/or evaluating pacing therapy at that location (e.g., the location of an implantable electrode used for pacing, the location of pacing therapy delivered by a particular pacing vector, etc.).

The computing apparatus **1140** may be operatively coupled to the input apparatus **1142** and the display apparatus **1130** to, e.g., transmit data to and from each of the input apparatus **1142** and the display apparatus **1130.** For example, the computing apparatus **1140** may be electrically coupled to each of the input apparatus **1142** and the display apparatus **1130** using, e.g., analog electrical connections, digital electrical connections, wireless connections, bus-based connections, network-based connections, internet-based connections, etc. As described further herein, a user may provide input to the input apparatus **1142** to manipulate, or modify, one or more graphical depictions displayed on the display apparatus **1130** and to view and/or select one or more pieces of information related to the cardiac therapy.

Although as depicted the input apparatus **1142** is a keyboard, it is to be understood that the input apparatus **1142** may include any apparatus capable of providing input to the computing apparatus **1140** to perform the functionality, methods, and/or logic described herein. For example, the input apparatus **1142** may include a mouse, a trackball, a touchscreen (e.g., capacitive touchscreen, a resistive touchscreen, a multi-touch touchscreen, etc.), etc. Likewise, the display apparatus **1130** may include any apparatus capable of displaying information to a user, such as a graphical user interface **1132** including cardiac information, textual instructions, graphical depictions of electrical activation information, graphical depictions of anatomy of a human heart, images or graphical depictions of the patient's heart, graphical depictions of a leadless and/or leaded pacing device being positioned or placed to provide VfA pacing therapy, graphical depictions of locations of one or more electrodes, graphical depictions of a human torso, images or graphical depictions of the patient's torso, graphical depictions or actual images of implanted electrodes and/or leads, etc. Further, the display apparatus **1130** may include a liquid crystal display, an organic light-emitting diode screen, a touchscreen, a cathode ray tube display, etc.

The processing programs or routines stored and/or executed by the computing apparatus **1140** may include programs or routines for computational mathematics, matrix mathematics, dispersion determinations (e.g., standard deviations, variances, ranges, interquartile ranges, mean absolute differences, average absolute deviations, etc.), filtering algorithms, maximum value determinations, minimum value determinations, threshold determinations, moving windowing algorithms, decomposition algorithms, compression algorithms (e.g., data compression algorithms), calibration algorithms, image construction algorithms, signal processing algorithms (e.g., various filtering algorithms, Fourier transforms, fast Fourier transforms, etc.), standardization algorithms, comparison algorithms, vector mathematics, or any other processing required to implement one or more illustrative methods and/or processes described herein. Data stored and/or used by the computing apparatus **1140** may include, for example, electrical signal/waveform data from the electrode apparatus **1110,** dispersions signals, windowed dispersions signals, parts or portions of various signals, electrical activation times from the electrode apparatus **1110,** graphics (e.g., graphical elements, icons, buttons, windows, dialogs, pull-down menus, graphic areas, graphic regions, 3D graphics, etc.), graphical user interfaces, results from one or more processing programs or routines employed according to the disclosure herein (e.g., electrical signals, cardiac information, etc.), or any other data that may be necessary for carrying out the one and/or more processes or methods described herein.

In one or more embodiments, the illustrative systems, methods, and interfaces may be implemented using one or more computer programs executed on programmable computers, such as computers that include, for example, processing capabilities, data storage (e.g., volatile or non-volatile memory and/or storage elements), input devices, and output devices. Program code and/or logic described herein may be applied to input data to perform functionality described herein and generate desired output information. The output information may be applied as input to one or more other devices and/or methods as described herein or as would be applied in a known fashion.

The one or more programs used to implement the systems, methods, and/or interfaces described herein may be provided using any programmable language, e.g., a high-level procedural and/or object orientated programming language that is suitable for communicating with a computer system. Any such programs may, for example, be stored on any suitable device, e.g., a storage media, that is readable by a general or special purpose program running on a computer system (e.g., including processing apparatus) for configuring and operating the computer system when the suitable device is read for performing the procedures described herein. In other words, at least in one embodiment, the illustrative systems, methods, and/or interfaces may be implemented using a computer readable storage medium, configured with a computer program, where the storage medium so configured causes the computer to operate in a specific and predefined manner to perform functions described herein. Further, in at least one embodiment, the illustrative systems, methods, and/or interfaces may be described as being implemented by logic (e.g., object code) encoded in one or more non-transitory media that includes code for execution and, when executed by a processor, is operable to perform operations such as the methods, processes, and/or functionality described herein.

The computing apparatus **1140** may be, for example, any fixed or mobile computer system (e.g., a controller, a microcontroller, a personal computer, minicomputer, tablet computer, etc.) and may be generally described as including processing circuitry. The exact configuration of the computing apparatus **1140** is not limiting, and essentially any device capable of providing suitable computing capabilities and control capabilities (e.g., graphics processing, etc.) may be used. As described herein, a digital file may be any medium (e.g., volatile or non-volatile memory, a CD-ROM, a punch card, magnetic recordable medium such as a disk or tape, etc.) containing digital bits (e.g., encoded in binary, trinary, etc.) that may be readable and/or writeable by computing apparatus **1140** described herein. Also, as described herein, a file in user-readable format may be any representation of data (e.g., ASCII text, binary numbers, hexadecimal numbers, decimal numbers, graphically, etc.) presentable on any medium (e.g., paper, a display, etc.) readable and/or understandable by a user.

In view of the above, it will be readily apparent that the functionality as described in one or more embodiments according to the present disclosure may be implemented in any manner as would be known to one skilled in the art. As such, the computer language, the computer system, or any other software/hardware which is to be used to implement the processes described herein shall not be limiting on the scope of the systems, processes or programs (e.g., the functionality provided by such systems, processes or programs) described herein.

Electrical activation times of the patient's heart may be useful to evaluate a patient's cardiac condition and/or ventricle from atrium (VfA) cardiac therapy being delivered to a patient. Surrogate electrical activation information or data of one or more regions of a patient's heart may be monitored, or determined, using electrode apparatus **1110** as shown in **FIG. 20** and in **FIGS. 21-22****.** The illustrative electrode apparatus **1110** may be configured to measure body-surface potentials of a patient **1120** and, more particularly, torso-surface potentials of a patient **1120.** As shown in **FIG. 21****,** the illustrative electrode apparatus **1110** may include a set, or array, of electrodes **1112,** a strap **1113,** and interface/amplifier circuitry **1116.** In at least one embodiment, a portion of the set of electrodes may be used wherein the portion corresponds to a particular location on the patient's heart. The electrodes **1112** may be attached, or coupled, to the strap **1113** and the strap **1113** may be configured to be wrapped around the torso of a patient **1120** such that the electrodes **1112** surround the patient's heart. As further illustrated, the electrodes **1112** may be positioned around the circumference of a patient **1120,** including the posterior, lateral, posterolateral, anterolateral, and anterior locations of the torso of a patient **1120.**

Further, the electrodes **1112** may be electrically connected to interface/amplifier circuitry **1116** via wired connection **1118.** The interface/amplifier circuitry **1116** may be configured to amplify the signals from the electrodes **1112** and provide the signals to the computing apparatus **1140.** Other illustrative systems may use a wireless connection to transmit the signals sensed by electrodes **1112** to the interface/amplifier circuitry **1116** and, in turn, the computing apparatus **1140,** e.g., as channels of data. For example, the interface/amplifier circuitry **1116** may be electrically coupled to each of the computing apparatus **1140** and the display apparatus **1130** using, e.g., analog electrical connections, digital electrical connections, wireless connections, bus-based connections, network-based connections, internet-based connections, etc.

Although in the example of **FIG. 21** the electrode apparatus **1110** includes a strap **1113,** in other examples any of a variety of mechanisms, e.g., tape or adhesives, may be employed to aid in the spacing and placement of electrodes **1112.** In some examples, the strap **1113** may include an elastic band, strip of tape, or cloth. In other examples, the electrodes **1112** may be placed individually on the torso of a patient **1120.** Further, in other examples, electrodes **1112** (e.g., arranged in an array) may be part of, or located within, patches, vests, and/or other manners of securing the electrodes **1112** to the torso of the patient **1120.**

The electrodes **1112** may be configured to surround the heart of the patient **1120** and record, or monitor, the electrical signals associated with the depolarization and repolarization of the heart after the signals have propagated through the torso of a patient **1120.** Each of the electrodes **1112** may be used in a unipolar configuration to sense the torso-surface potentials that reflect the cardiac signals. The interface/amplifier circuitry **1116** may also be coupled to a return or indifferent electrode (not shown) that may be used in combination with each electrode **1112** for unipolar sensing. In some examples, there may be about 12 to about 50 electrodes **1112** spatially distributed around the torso of patient. Other configurations may have more or fewer electrodes **1112.**

The computing apparatus **1140** may record and analyze the electrical activity (e.g., torso-surface potential signals) sensed by electrodes **1112** and amplified/conditioned by the interface/amplifier circuitry **1116.** The computing apparatus **1140** may be configured to analyze the signals from the electrodes **1112** to provide as anterior and posterior electrode signals and surrogate cardiac electrical activation times, e.g., representative of actual, or local, electrical activation times of one or more regions of the patient's heart as will be further described herein. The computing apparatus **1140** may be configured to analyze the signals from the electrodes **1112** to provide as anterior-septal electrode signals and surrogate cardiac electrical activation times, e.g., representative of actual, or local, electrical activation times of one or more anterior-septal regions of the patient's heart, as will be further described herein, e.g., for use in evaluation of VfA pacing therapy. Further, the electrical signals measured at the left anterior surface location of a patient's torso may be representative, or surrogates, of electrical signals of the left anterior left ventricle region of the patient's heart, electrical signals measured at the left lateral surface location of a patient's torso may be representative, or surrogates, of electrical signals of the left lateral left ventricle region of the patient's heart, electrical signals measured at the left posterolateral surface location of a patient's torso may be representative, or surrogates, of electrical signals of the posterolateral left ventricle region of the patient's heart, and electrical signals measured at the posterior surface location of a patient's torso may be representative, or surrogates, of electrical signals of the posterior left ventricle region of the patient's heart. In one or more embodiments, measurement of activation times can be performed by measuring the period of time between an onset of cardiac depolarization (e.g., onset of QRS complex) and an appropriate fiducial point such as, e.g., a peak value, a minimum value, a minimum slope, a maximum slope, a zero crossing, a threshold crossing, etc.

Additionally, the computing apparatus **1140** may be configured to provide graphical user interfaces depicting the surrogate electrical activation times obtained using the electrode apparatus **1110.** Illustrative systems, methods, and/or interfaces may noninvasively use the electrical information collected using the electrode apparatus **1110** to evaluate a patient's cardiac condition and/or ventricle from atrium pacing therapy being delivered to the patient.

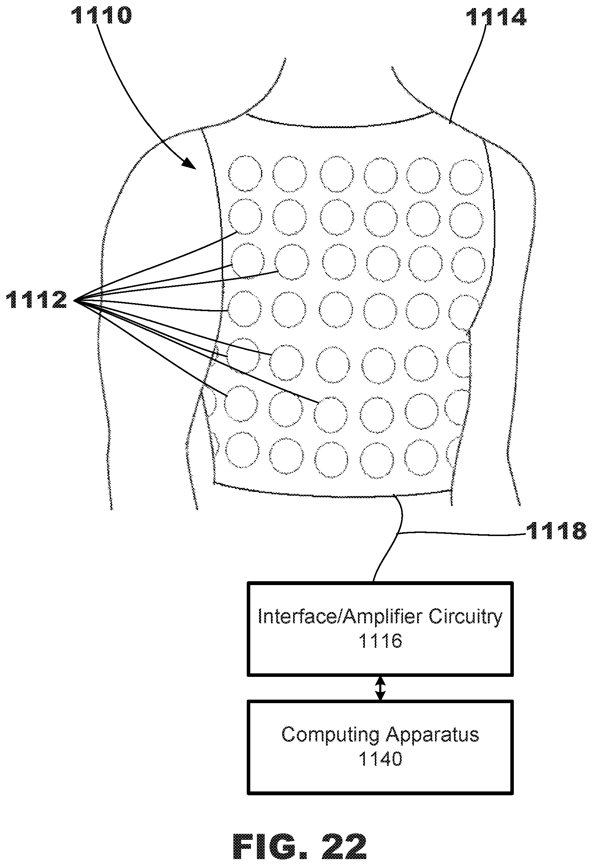

**FIG. 22** illustrates another illustrative electrode apparatus **1110** that includes a plurality of electrodes **1112** configured to surround the heart of the patient **1120** and record, or monitor, the electrical signals associated with the depolarization and repolarization of the heart after the signals have propagated through the torso of the patient **1120.** The electrode apparatus **1110** may include a vest **1114** upon which the plurality of electrodes **1112** may be attached, or to which the electrodes **1112** may be coupled. In at least one embodiment, the plurality, or array, of electrodes **1112** may be used to collect electrical information such as, e.g., surrogate electrical activation times. Similar to the electrode apparatus **1110** of **FIG. 21****,** the electrode apparatus **1110** of **FIG. 22** may include interface/amplifier circuitry **1116** electrically coupled to each of the electrodes **1112** through a wired connection **1118** and be configured to transmit signals from the electrodes **1112** to computing apparatus **1140.** As illustrated, the electrodes **1112** may be distributed over the torso of a patient **1120,** including, for example, the anterior, lateral, posterolateral, anterolateral, and posterior surfaces of the torso of the patient **1120.**

The vest **1114** may be formed of fabric with the electrodes **1112** attached to the fabric. The vest **1114** may be configured to maintain the position and spacing of electrodes **1112** on the torso of the patient **1120.** Further, the vest **1114** may be marked to assist in determining the location of the electrodes **1112** on the surface of the torso of the patient **1120.** In one or more embodiments, the vest **1114** may include 17 or more anterior electrodes positionable proximate the anterior torso of the patient, and 39 or more posterior electrodes positionable proximate the anterior torso of the patient. In some examples, there may be about 25 electrodes **1112** to about 256 electrodes **1112** distributed around the torso of the patient **1120,** though other configurations may have more or less electrodes **1112.**

As described herein, the electrode apparatus **1110** may be configured to measure electrical information (e.g., electrical signals) representing different regions of a patient's heart. For example, activation times of different regions of a patient's heart can be approximated from surface electrocardiogram (ECG) activation times measured using surface electrodes in proximity to surface areas corresponding to the different regions of the patient's heart. In at least one example, activation times of the anterior-septal region of a patient's heart can be approximated from surface ECG activation times measured using surface electrodes in proximity to surface areas corresponding to the anterior-septal region of the patient's heart. That is, a portion of the set of electrodes **1112,** and not the entire set, can be used to generate activation times corresponding to a particular location of the patient's heart that the portion of the set of electrodes corresponds to.

The illustrative systems, methods, and interfaces may be used to provide noninvasive assistance to a user in the evaluation of a patient's cardiac health or status, and/or the evaluation of cardiac therapy such as ventricle from atrium (VfA) pacing therapy by use of the electrode apparatus **1110** (e.g., cardiac therapy being presently-delivered to a patient during implantation or after implantation). Further, the illustrative systems, methods, and interfaces may be used to assist a user in the configuration of the cardiac therapy, such as VfA pacing therapy, being delivered to a patient.

VfA pacing can be described as providing a synchronized homogeneous activation of ventricles of the heart. As an example, patients with atrial-ventricular (AV) block or prolonged AV timings that can lead to heart failure who have otherwise intact (e.g., normal) QRS can benefit from VfA pacing therapy. In addition, as an example, VfA pacing may provide beneficial activation for heart failure patients with intrinsic ventricular conduction disorders. Further, proper placement of VfA pacing can provide optimal activation of the ventricles for such patients. Further, left ventricular (LV) resynchronization for heart failure patients with left bundle branch block (LBBB) may find that VfA pacing enables easier access to left ventricular endocardium without exposing the leadless device or lead to endocardial blood pool. At the same time, in that example, this can help engage part of the conduction system to potentially correct LBBB and effectively resynchronize the patient.

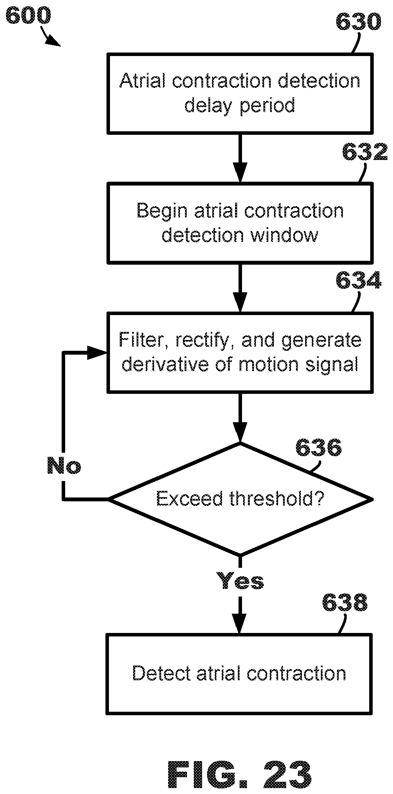

**FIG. 23** shows an illustrative method **600** of detecting atrial activity, for example, using the motion detector **11** of **FIG. 2****.** In particular, method **600** may include detecting an atrial contraction based on analysis of a motion signal (e.g., provided by the motion detector **11)** that may be performed by an IMD implanted in the patient's heart. In some embodiments, the motion signal may be provided by an IMD implanted within a ventricle, such as the right ventricle, of the patient's heart. The method **600** may include beginning an atrial contraction detection delay period upon identification of a ventricular activation event **630.** The method **600** may include beginning an atrial contraction detection window upon expiration of the atrial contraction delay period **632.** The method **600** may include analyzing the motion signal within the atrial contraction detection window.

The method **600** may include filtering the motion signal within the atrial contraction detection window, rectifying the filtered signal, and generating a derivative signal of the filtered and rectified motion signal within the atrial contraction detection window **634.** The method **600** may include determining whether an amplitude of the derivative signal within the atrial contraction detection window exceeds a threshold **636.** In response to determining that the amplitude of the derivative signal within the atrial contraction detection window exceeds the threshold (YES of **636),** the method **600** may proceed to detecting atrial contraction **638.** Otherwise (NO of **636),** the method **600** may return to filtering, rectifying, and generating a derivative signal **634.** Various techniques for using a motion detector that provides a motion signal may be described in U.S. Patent No. 9,399,140 (Cho et al.), issued July 26, 2016, entitled "Atrial contraction detection by a ventricular leadless pacing device for atrio-synchronous ventricular pacing," which is incorporated herein by reference in its entirety.

### ILLUSTRATIVE EMBODIMENTS

While the present disclosure is not so limited, an appreciation of various aspects of the disclosure of the present application will be gained through a discussion of some illustrative embodiments provided below.

Various illustrative embodiments are directed to atrioventricular synchronous pacing.

In illustrative embodiment 1, an implantable medical device includes a plurality of electrodes. The plurality of electrodes includes a tissue-piercing electrode implantable from the triangle of Koch region of the right atrium through the right atrial endocardium and central fibrous body to at least one of deliver cardiac therapy to and sense electrical activity of the left ventricle in the basal and/or septal region of the left ventricular myocardium of a patient's heart. The plurality of electrodes also includes a right atrial electrode positionable within the right atrium to at least one of deliver cardiac therapy and sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to monitor electrical activity of the right atrium using the right atrial electrode and deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity of the right atrium.

In illustrative embodiment 2, a method includes monitoring activity of the right atrium of a patient's heart using a right atrial electrode or a right atrial motion detector and delivering atrioventricular synchronous pacing using at least a tissue-piercing electrode implanted in the basal and/or septal region of the left ventricular myocardium of the patient's heart from the triangle of Koch region of the right atrium through the right atrial endocardium and central fibrous body including pacing one or both ventricles using at least the tissue-piercing electrode based on the monitored activity of the right atrium.

In illustrative embodiment 3, a device or method of any preceding illustrative embodiment is included, wherein delivering atrioventricular synchronous pacing includes using the right atrial electrode to pace the right atrium.

In illustrative embodiment 4, a device or method of any preceding illustrative embodiment is included, wherein delivering atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity of the right atrium includes sensing an atrial event within the monitored electrical activity of the right atrium and delivering a pace to one or both ventricles in response to the sensed atrial event.

In illustrative embodiment 5, a device or method of illustrative embodiment 4 is included, wherein delivering the pace to one or both ventricles in response to the sensed atrial event includes delivering the pace to one or both ventricles after a selected A-V time period has elapsed from the sensed atrial event.

In illustrative embodiment 6, a device or method of any preceding illustrative embodiment is included, wherein the controller is further configured to perform, or the method further includes, monitoring electrical activity of the left ventricle in the basal and/or septal region of the left ventricular myocardium using the tissue-piercing electrode implanted in the basal and/or septal region of the left ventricular myocardium of the patient's heart from the triangle of Koch region of the right atrium through the right atrial endocardium and central fibrous body; sensing a ventricular event within the monitored electrical activity from using the tissue-piercing electrode; and withholding delivering the pace to one or both ventricles in response to sensing the ventricular event within the monitored electrical activity from using the tissue-piercing electrode.

In illustrative embodiment 7, a device or method of any preceding illustrative embodiment is included, wherein the controller is further configured to perform, or the method further includes, detecting atrial fibrillation and initiating a single chamber pacing mode.

In illustrative embodiment 8, a device or method of any preceding illustrative embodiment is included, wherein the controller is further configured to perform, or the method further includes, receiving a trigger from a separate medical device in a triggered pacing mode.

In illustrative embodiment 8, a device or method of any preceding illustrative embodiment is included, wherein the controller is further configured to perform, or the method further includes, delivering pacing in an asynchronous pacing mode.

In illustrative embodiment 9, a device of any preceding illustrative embodiment is included, further including a housing extending from a proximal end region to a distal end region. The right atrial electrode is leadlessly coupled to the housing and the tissue-piercing electrode is leadlessly coupled to the distal end region of the housing. The therapy delivery circuit, the sensing circuit, and the controller are located within the housing.

In illustrative embodiment 10, the device of illustrative embodiment 9 is included, further including a fixation member that extends from the housing. The fixation member is configured to pierce into the myocardium. The fixation member extends from the distal end region of the housing toward a distal tip of the tissue-piercing electrode.

In illustrative embodiment 11, a device of any preceding illustrative embodiment is included, further including a housing, wherein the therapy delivery circuit, the sensing circuit, and the controller are located within the housing. The device further includes a lead coupled to and extending from the housing to a distal end region. The tissue-piercing electrode and the right atrial electrode are coupled to the distal end region of the lead.

In illustrative embodiment 12, a device of any preceding illustrative embodiment is included, further including a housing, wherein the therapy delivery circuit, the sensing circuit, and the controller are located within the housing. The device further includes a first lead coupled to and extending from the housing to a first distal end region and a second lead coupled to and extending from the housing to a second distal end region. The tissue-piercing electrode is coupled to the first distal end region of the first lead. The right atrial electrode is coupled to the second distal end region of the second lead.

In illustrative embodiment 13, a device of any preceding illustrative embodiment is included, further including a housing, wherein the therapy delivery circuit, the sensing circuit, and the controller are located within the housing. The device further includes a first lead coupled to and extending from the housing to a first distal end region, a second lead coupled to and extending from the housing to a second distal end region, and a third lead coupled to and extending from the housing to a third distal end region. The plurality of electrodes further includes a right ventricular electrode to deliver cardiac therapy to or sense electrical activity of the right ventricle of the patient's heart coupled to the third distal end region of the third lead. The tissue-piercing electrode is coupled to the first distal end region of the first lead. The right atrial electrode is coupled to the second distal end region of the second lead.

In illustrative embodiment 14, an implantable medical device includes a housing extending from a proximal end region to a distal end region. The device also includes a plurality of electrodes. The plurality of electrodes includes a tissue-piercing electrode leadlessly coupled to the distal end region of the housing and implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode leadlessly coupled to the housing and positionable within the right atrium to deliver cardiac therapy to or sense electrical activity of the right atrium of the patient's heart. The implantable medical device also includes a therapy delivery circuit within the housing operably coupled to the plurality of electrodes to deliver cardiac therapy to the patient's heart and a sensing circuit within the housing operably coupled to the plurality of electrodes to sense electrical activity of the patient's heart. The implantable medical device further includes a controller including processing circuitry within the housing operably coupled to the therapy delivery circuit and the sensing circuit. The controller is configured to monitor electrical activity of the right atrium using the right atrial electrode and deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity of the right atrium.

In illustrative embodiment 15, an implantable medical device includes a plurality of electrodes. The plurality of electrodes includes a tissue-piercing electrode implantable from the triangle of Koch region of the right atrium through the right atrial endocardium and central fibrous body to at least one of deliver cardiac therapy to and sense electrical activity of the left ventricle in the basal and/or septal region of the left ventricular myocardium of a patient's heart. The tissue-piercing electrode also includes a right atrial motion detector positionable within the right atrium to sense mechanical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to monitor mechanical activity of the right atrium using the right atrial motion detector and deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored mechanical activity of the right atrium.

Various illustrative embodiments are directed to cardiac resynchronization pacing.

In illustrative embodiment 16, an implantable medical device includes a plurality of electrodes. The plurality of electrodes includes a tissue-piercing electrode implantable from the triangle of Koch region of the right atrium through the right atrial endocardium and central fibrous body to at least one of deliver cardiac therapy to or sense electrical activity of the left ventricle in the basal and/or septal region of the left ventricular myocardium of a patient's heart. The plurality of electrodes also includes a right atrial electrode positionable within the right atrium to at least one of deliver cardiac therapy to or sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to monitor at least one of electrical activity of the right atrium using the right atrial electrode and electrical activity of the left ventricle using the tissue-piercing electrode implanted in the basal and/or septal region of the left ventricular myocardium of the patient's heart from the triangle of Koch region of the right atrium through the right atrial endocardium and central fibrous body. The controller is also configured to deliver cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity.

In illustrative embodiment 17, a method includes monitoring at least one of electrical activity of the right atrium of a patient's heart using a right atrial electrode and electrical activity of the left ventricle using a tissue-piercing electrode implanted in the basal and/or septal region of the left ventricular myocardium of the patient's heart from the triangle of Koch region of the right atrium through the right atrial endocardium and central fibrous body. The method also includes delivering cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body including pacing one or both ventricles based on the monitored electrical activity.

In illustrative embodiment 18, a device or method of any preceding illustrative embodiment is included, wherein the controller is further configured to perform, or the method further includes, monitoring far-field electrical activity of the right ventricle using the right atrial electrode.

In illustrative embodiment 19, a device or method of any illustrative embodiment 16-18 is included, wherein delivering cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity includes sensing an atrial event within the monitored electrical activity of the right atrium and delivering a pace to one or both ventricles in response to the sensed atrial event to provide cardiac resynchronization.

In illustrative embodiment 20, a device or method of any illustrative embodiment 16-19 is included, wherein delivering cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity includes sensing a ventricular event within the monitored electrical activity from using the tissue-piercing electrode and delivering a pace to one or both ventricles in response to the sensed ventricular event to provide cardiac resynchronization.

In illustrative embodiment 21, the device or method of illustrative embodiment 20 is included, wherein delivering a pace to one or both ventricles in response to the sensed ventricular event includes delivering the pace to one or both ventricles after a selected time period has elapsed from the sensed ventricular event.

In illustrative embodiment 22, a device or method of any illustrative embodiment 16-21 is included, wherein the controller is further configured to perform, or the method further includes, detecting atrial fibrillation and initiating a single chamber pacing mode.

In illustrative embodiment 23, a device or method of any illustrative embodiment 16-22 is included, wherein the controller is further configured to perform, or the method further includes, receiving a trigger from a separate medical device in a triggered pacing mode.

In illustrative embodiment 24, a device or method of any illustrative embodiment 16-23 is included, wherein the controller is further configured to perform, or the method further includes, delivering pacing in an asynchronous pacing mode.

In illustrative embodiment 25, a device of any illustrative embodiment 16-24 is included, further including a housing extending from a proximal end region to a distal end region. The right atrial electrode is leadlessly coupled to the housing and the tissue-piercing electrode is leadlessly coupled to the distal end region of the housing. The therapy delivery circuit, the sensing circuit, and the controller are located within the housing.

In illustrative embodiment 26, the device of illustrative embodiment 25 is included, further including a fixation member that extends from the housing. The fixation member is configured to pierce into the myocardium. The fixation member extends from the distal end region of the housing toward a distal tip of the tissue-piercing electrode.

In illustrative embodiment 27, a device of any illustrative embodiment 16-26 is included, further including a housing, wherein the therapy delivery circuit, the sensing circuit, and the controller are located within the housing. The device further includes a lead coupled to and extending from the housing to a distal end region. The tissue-piercing electrode and the right atrial electrode are coupled to the distal end region of the lead.

In illustrative embodiment 28, a device of any illustrative embodiment 16-27 is included, further including a housing, wherein the therapy delivery circuit, the sensing circuit, and the controller are located within the housing. The device further includes a first lead coupled to and extending from the housing to a first distal end region and a second lead coupled to and extending from the housing to a second distal end region. The tissue-piercing electrode is coupled to the first distal end region of the first lead. The right atrial electrode is coupled to the second distal end region of the second lead.

In illustrative embodiment 29, a device of any illustrative embodiment 16-28 is included, further including a housing, wherein the therapy delivery circuit, the sensing circuit, and the controller are located within the housing. The device further includes a first lead coupled to and extending from the housing to a first distal end region, a second lead coupled to and extending from the housing to a second distal end region, and a third lead coupled to and extending from the housing to a third distal end region. The plurality of electrodes further includes a right ventricular electrode to deliver cardiac therapy to or sense electrical activity of the right ventricle of the patient's heart coupled to the third distal end region of the third lead. The tissue-piercing electrode is coupled to the first distal end region of the first lead. The right atrial electrode is coupled to the second distal end region of the second lead.

In illustrative embodiment 30, an implantable medical device includes a housing extending from a proximal end region to a distal end region. The implantable medical device also includes a plurality of electrodes. The plurality of electrodes includes a tissue-piercing electrode leadlessly coupled to the distal end region of the housing and implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode leadlessly coupled to the housing and positionable within the right atrium to deliver cardiac therapy to or sense electrical activity of the right atrium of the patient's heart. The implantable medical device also includes a therapy delivery circuit within the housing operably coupled to the plurality of electrodes to deliver cardiac therapy to the patient's heart and a sensing circuit within the housing operably coupled to the plurality of electrodes to sense electrical activity of the patient's heart. The implantable medical device further includes a controller including processing circuitry within the housing operably coupled to the therapy delivery circuit and the sensing circuit. The controller is configured to monitor at least one of electrical activity of the right atrium using the right atrial electrode and electrical activity of the left ventricle using the tissue-piercing electrode in the basal and/or septal region of the left ventricular myocardium of the patient's heart from the triangle of Koch region of the right atrium through the right atrial endocardium and central fibrous body. The controller is further configured to deliver cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity.

In illustrative embodiment 31, an implantable medical device includes a plurality of electrodes. The plurality of electrodes includes a tissue-piercing electrode implantable from the triangle of Koch region of the right atrium through the right atrial endocardium and central fibrous body to at least one of deliver cardiac therapy to and sense electrical activity of the left ventricle in the basal and/or septal region of the left ventricular myocardium of a patient's heart. The plurality of electrodes also includes a right atrial motion detector positionable within the right atrium to sense mechanical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to monitor mechanical activity of the right atrium using the right atrial motion detector and deliver cardiac resynchronization pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored mechanical activity of the right atrium.

Various illustrative embodiments are directed to tachycardia therapy.

In illustrative embodiment 32, an implantable medical device includes a plurality of electrodes. The plurality of electrodes includes a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode positionable within the right atrium to deliver cardiac therapy to or sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to: monitor electrical activity of the right atrium using the right atrial electrode; monitor electrical activity of the left ventricle using the tissue-piercing electrode implanted in the basal and/or septal region of the left ventricular myocardium of the patient's heart from the triangle of Koch region of the right atrium through the right atrial endocardium and central fibrous body to sense electrical activity of the left ventricle in the basal and/or septal region of the left ventricular myocardium; and deliver tachycardia therapy based on the monitored electrical activities of the right atrium and the left ventricle.

In illustrative embodiment 33, a method includes monitoring electrical activity of the right atrium of a patient's heart using a right atrial electrode; monitoring electrical activity of the left ventricle of the patient's heart using a tissue-piercing electrode implanted in the basal and/or septal region of the left ventricular myocardium of the patient's heart from the triangle of Koch region of the right atrium through the right atrial endocardium and central fibrous body to sense electrical activity of the left ventricle in the basal and/or septal region of the left ventricular myocardium; and deliver tachycardia therapy based on the monitored electrical activities of the right atrium and the left ventricle.

In illustrative embodiment 34, a device or method of any illustrative embodiment 32-33 is included, wherein the controller is further configured to perform, or the method further includes, delivering tachycardia therapy based on the monitored electrical activities of the right atrium and left ventricle includes delivering at least one of anti-tachycardia pacing therapy using the plurality of electrodes and shock therapy using a separate medical device.

In illustrative embodiment 35, a device or method of any illustrative embodiment 32-34 is included, wherein the controller is further configured to perform, or the method further includes, withholding tachycardia therapy in response to a determining that a cardiac rhythm of the patient's heart has 1: 1 atrioventricular conduction.

In illustrative embodiment 36, a device of any illustrative embodiment 32-35 is included, further including a housing extending from a proximal end region to a distal end region, wherein the right atrial electrode is leadlessly coupled to the housing and the tissue-piercing electrode is leadlessly coupled to the distal end region of the housing, wherein the therapy delivery circuit, the sensing circuit, and the controller are enclosed within the housing.

In illustrative embodiment 37, the device of illustrative embodiment 36 is included, further including a fixation member that extends from the housing. The fixation member is configured to pierce into the myocardium. The fixation member extends from the distal end region of the housing toward a distal tip of the tissue-piercing electrode.

In illustrative embodiment 38, a device of any illustrative embodiment 32-37 is included, further including a housing, wherein the therapy delivery circuit, the sensing circuit, and the controller are located within the housing. The device further includes a lead coupled to and extending from the housing to a distal end region. The tissue-piercing electrode and the right atrial electrode are coupled to the distal end region of the lead.

In illustrative embodiment 39, a device of any illustrative embodiment 32-38 is included, further including a housing, wherein the therapy delivery circuit, the sensing circuit, and the controller are located within the housing. The device further includes a first lead coupled to and extending from the housing to a first distal end region and a second lead coupled to and extending from the housing to a second distal end region. The tissue-piercing electrode is coupled to the first distal end region of the first lead. The right atrial electrode is coupled to the second distal end region of the second lead.

In illustrative embodiment 40, a device of any illustrative embodiment 32-39 is included, further including a housing, wherein the therapy delivery circuit, the sensing circuit, and the controller are located within the housing. The device further includes a first lead coupled to and extending from the housing to a first distal end region, a second lead coupled to and extending from the housing to a second distal end region, and a third lead coupled to and extending from the housing to a third distal end region. The plurality of electrodes further includes a right ventricular electrode to deliver cardiac therapy to or sense electrical activity of the right ventricle of the patient's heart coupled to the third distal end region of the third lead. The tissue-piercing electrode is coupled to the first distal end region of the first lead. The right atrial electrode is coupled to the second distal end region of the second lead.

In illustrative embodiment 41, an implantable medical device includes a plurality of electrodes. The plurality of electrodes includes a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode positionable within the right atrium to deliver cardiac therapy to or sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to: monitor electrical activity of the right atrium using the right atrial electrode; monitor electrical activity of the left ventricle using the tissue-piercing electrode implanted to sense electrical activity of the left ventricle in the basal and/or septal region of the left ventricular myocardium of the patient's heart from the triangle of Koch region of the right atrium through the right atrial endocardium and central fibrous body; and determine tachycardia of the patient's heart based on the monitored electrical activities of the right atrium and the left ventricle.

In illustrative embodiment 42, a method includes monitoring electrical activity of the right atrium of a patient's heart using a right atrial electrode; monitoring electrical activity of the left ventricle of the patient's heart using a tissue-piercing electrode implanted to sense electrical activity of the left ventricle in the basal and/or septal region of the left ventricular myocardium of the patient's heart from the triangle of Koch region of the right atrium through the right atrial endocardium and central fibrous body; and determining tachycardia of the patient's heart based on the monitored electrical activities of the right atrium and the left ventricle.

In illustrative embodiment 43, a device or method of any illustrative embodiment 41-42 is included, wherein the controller is further configured to perform, or the method further includes, determining whether a cardiac rhythm of the patient's heart has 1: 1 atrioventricular conduction.

In illustrative embodiment 44, a device or method of illustrative embodiment 43 is included, wherein the controller is further configured to perform, or the method further includes, determining appropriate tachycardia therapy based on the monitored electrical activities of the right atrium and the left ventricle in response to the cardiac rhythm not having 1: 1 atrioventricular conduction.

In illustrative embodiment 45, a device or method of any illustrative embodiment 41-44 is included, wherein determining tachycardia of the patient's heart based on the monitored electrical activities of the right atrium and the left ventricle includes determining that an atrial rhythm is regular and that a ventricular rhythm exceeds a selected ventricular rhythm threshold.

In illustrative embodiment 46, a device or method of any illustrative embodiment 41-45 is included, wherein the controller is further configured to perform, or the method further includes, delivering tachycardia therapy in response to determining tachycardia of the patient's heart.

In illustrative embodiment 47, a device or method of illustrative embodiment 46 is included, wherein the controller is further configured to perform, or the method further includes, delivering anti-tachycardia pacing therapy in response to a regular V-V event interval and a regular electrogram morphology.

In illustrative embodiment 48, a device or method of any illustrative embodiment 46-47 is included, wherein the controller is further configured to perform, or the method further includes, delivering shock therapy using a separate medical device in response to at least one of an irregular V-V event interval and an irregular electrogram morphology.

In illustrative embodiment 49, an implantable medical device includes a plurality of electrodes. The plurality of electrodes includes a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart. The plurality of electrodes also includes a right atrial electrode positionable within the right atrium to deliver cardiac therapy to or sense electrical activity of the right atrium of the patient's heart. The implantable medical device also includes a therapy delivery circuit operably coupled to the plurality of electrodes to deliver cardiac therapy to the patient's heart and a sensing circuit operably coupled to the plurality of electrodes to sense electrical activity of the patient's heart. The implantable medical device further includes a controller including processing circuitry operably coupled to the therapy delivery circuit and the sensing circuit. The controller is configured to deliver at least one of anti-tachycardia pacing therapy using the plurality of electrodes and shock therapy using a separate medical device.

In illustrative embodiment 50, a method includes delivering at least one of anti-tachycardia pacing therapy and shock therapy using a separate medical device using at least a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart.

In illustrative embodiment 51, a device or method of any illustrative embodiment 49-59 is included, wherein the controller is further configured to perform, or the method further includes, detecting whether a ventricular rhythm exceeds a selected ventricular rhythm threshold after beginning to deliver anti-tachycardia pacing therapy.

In illustrative embodiment 52, a device or method of any illustrative embodiment 49-51 is included, wherein the controller is further configured to perform, or the method further includes, monitoring for tachycardia in response to detecting a regular ventricular rhythm.

In illustrative embodiment 53, a device or method of any illustrative embodiment 49-52 is included, wherein the controller is further configured to perform, or the method further includes, delivering shock therapy using a separate device therapy in response to detecting a ventricular rhythm that exceeds the selected ventricular rhythm threshold.

In illustrative embodiment 54, a device or method of any illustrative embodiment 49-53 is included, wherein the shock therapy includes delivering a signaling pulse to the left ventricle using the tissue-piercing electrode.

In illustrative embodiment 55, a device or method of illustrative embodiment 54 is included, wherein the signaling pulse is configured to be detected by the separate medical device to trigger delivery of a shock from the separate medical device to the cardiac tissue.

In illustrative embodiment 56, a device or method of any illustrative embodiment 49-54 is included, wherein the separate medical device is not connected to the implantable medical device by a metal conductor.

Thus, various embodiments of VFA CARDIAC THERAPY are disclosed. Although reference is made herein to the accompanying set of drawings that form part of this disclosure, one of at least ordinary skill in the art will appreciate that various adaptations and modifications of the embodiments described herein are within, or do not depart from, the scope of this disclosure. For example, aspects of the embodiments described herein may be combined in a variety of ways with each other. Therefore, it is to be understood that, within the scope of the appended claims, the claimed invention may be practiced other than as explicitly described herein.

It will be understood that each block of the block diagrams and combinations of those blocks can be implemented by means for performing the illustrated function.

All references and publications cited herein are expressly incorporated herein by reference in their entirety into this disclosure, except to the extent they may directly contradict this disclosure.

All scientific and technical terms used herein have meanings commonly used in the art unless otherwise specified. The definitions provided herein are to facilitate understanding of certain terms used frequently herein and are not meant to limit the scope of the present disclosure.

Unless otherwise indicated, all numbers expressing feature sizes, amounts, and physical properties used in the specification and claims may be understood as being modified either by the term "exactly" or "about." Accordingly, unless indicated to the contrary, the numerical parameters set forth in the foregoing specification and attached claims are approximations that can vary depending upon the desired properties sought to be obtained by those skilled in the art utilizing the teachings disclosed herein or, for example, within typical ranges of experimental error.

The recitation of numerical ranges by endpoints includes all numbers subsumed within that range (e.g., 1 to 5 includes 1, 1.5, 2, 2.75, 3, 3.80, 4, and 5) and any range within that range. Herein, the terms "up to" or "no greater than" a number (e.g., up to 50) includes the number (e.g., 50), and the term "no less than" a number (e.g., no less than 5) includes the number (e.g., 5).

The terms "coupled" or "connected" refer to elements being attached to each other either directly (in direct contact with each other) or indirectly (having one or more elements between and attaching the two elements). Either term may be modified by "operatively" and "operably," which may be used interchangeably, to describe that the coupling or connection is configured to allow the components to interact to carry out at least some functionality (for example, an intracardiac medical device may be operatively or operably coupled to an extravascular ICD to initiate shock therapy).

Reference to "one embodiment," "an embodiment," "certain embodiments," or "some embodiments," etc., means that a particular feature, configuration, composition, or characteristic described in connection with the embodiment is included in at least one embodiment of the disclosure. Thus, the appearances of such phrases in various places throughout are not necessarily referring to the same embodiment of the disclosure. Furthermore, the particular features, configurations, compositions, or characteristics may be combined in any suitable manner in one or more embodiments.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure.

As used in this specification and the appended claims, the singular forms "a," "an," and "the" encompass embodiments having plural referents, unless the content clearly dictates otherwise. As used in this specification and the appended claims, the term "or" is generally employed in its sense including "and/or" unless the content clearly dictates otherwise.

As used herein, "have," "having," "include," "including," "comprise," "comprising" or the like are used in their open-ended sense, and generally mean "including, but not limited to." It will be understood that "consisting essentially of," "consisting of," and the like are subsumed in "comprising," and the like.

The term "and/or" means one or all of the listed elements or a combination of at least two of the listed elements.

The phrases "at least one of," "comprises at least one of," and "one or more of" followed by a list refers to any one of the items in the list and any combination of two or more items in the list.

Further examples, which can be combined with other examples described herein, are described in the following clauses. Any features described as part of one these clauses can be used on or in conjunction with other examples described herein to yield yet a further example.
1. An implantable medical device comprising:
   a plurality of electrodes comprising:
      a tissue-piercing electrode implantable from the triangle of Koch region of the right atrium through the right atrial endocardium and central fibrous body to at least one of deliver cardiac therapy to and sense electrical activity of the left ventricle in the basal and/or septal region of the left ventricular myocardium of a patient's heart, and
      a right atrial electrode positionable within the right atrium to at least one of deliver cardiac therapy and sense electrical activity of the right atrium of the patient's heart;
   a therapy delivery circuit operably coupled to the plurality of electrodes to deliver cardiac therapy to the patient's heart;
   a sensing circuit operably coupled to the plurality of electrodes to sense electrical activity of the patient's heart; and
   a controller comprising processing circuitry operably coupled to the therapy delivery circuit and the sensing circuit, the controller configured to:
      monitor electrical activity of the right atrium using the right atrial electrode; and
      deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity of the right atrium.
2. A method comprising:
   monitoring activity of the right atrium of a patient's heart using a right atrial electrode or a right atrial motion detector; and
   delivering atrioventricular synchronous pacing using at least a tissue-piercing electrode implanted in the basal and/or septal region of the left ventricular myocardium of the patient's heart from the triangle of Koch region of the right atrium through the right atrial endocardium and central fibrous body comprising pacing one or both ventricles using at least the tissue-piercing electrode based on the monitored activity of the right atrium.
3. The device according to clause 1 or the method according to claim 2, wherein delivering atrioventricular synchronous pacing further comprises using the right atrial electrode to pace the right atrium.
4. The device or method according to any preceding clause, wherein delivering atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity of the right atrium comprises:
   sensing an atrial event within the monitored electrical activity of the right atrium; and
   delivering a pace to one or both ventricles in response to the sensed atrial event.
5. The device or method according to clause 4, wherein delivering the pace to one or both ventricles in response to the sensed atrial event comprises delivering the pace to one or both ventricles after a selected A-V time period has elapsed from the sensed atrial event.
6. The device or method according to any preceding clause, wherein the controller is further configured to perform, or the method further comprises:
   monitoring electrical activity of the left ventricle in the basal and/or septal region of the left ventricular myocardium using the tissue-piercing electrode implanted in the basal and/or septal region of the left ventricular myocardium of the patient's heart from the triangle of Koch region of the right atrium through the right atrial endocardium and central fibrous body;
   sensing a ventricular event within the monitored electrical activity from using the tissue-piercing electrode; and
   withholding delivering the pace to one or both ventricles in response to sensing the ventricular event within the monitored electrical activity from using the tissue-piercing electrode.
7. The device or method according to any preceding clause, wherein the controller is further configured to perform, or the method further comprises, detecting atrial fibrillation and initiate a single chamber pacing mode.
8. The device or method according to any preceding clause, wherein the controller is further configured to perform, or the method further comprises, receiving a trigger from a separate medical device in a triggered pacing mode.
9. The device or method according to any preceding clause, wherein the controller is further configured to perform, or the method further comprises, delivering pacing in an asynchronous pacing mode.
10. The device according to any preceding clause, further comprising:
   a housing extending from a proximal end region to a distal end region, wherein the right atrial electrode is leadlessly coupled to the housing and the tissue-piercing electrode is leadlessly coupled to the distal end region of the housing, wherein the therapy delivery circuit, the sensing circuit, and the controller are located within the housing; and
   optionally a fixation member that extends from the housing, the fixation member being configured to pierce into the myocardium, wherein the fixation member extends from the distal end region of the housing toward a distal tip of the tissue-piercing electrode.
11. The device according to any one of clauses 1-9, further comprising:
   a housing, wherein the therapy delivery circuit, the sensing circuit, and the controller are located within the housing; and
   a lead coupled to and extending from the housing to a distal end region, wherein the tissue-piercing electrode and the right atrial electrode are coupled to the distal end region of the lead.
12. The device according to any one of clauses 1-9, further comprising:
   a housing, wherein the therapy delivery circuit, the sensing circuit, and the controller are located within the housing;
   a first lead coupled to and extending from the housing to a first distal end region; and
   a second lead coupled to and extending from the housing to a second distal end region, wherein the tissue-piercing electrode is coupled to the first distal end region of the first lead, wherein the right atrial electrode is coupled to the second distal end region of the second lead.
13. The device according to any one of clauses 1-9, further comprising:
   a housing, wherein the therapy delivery circuit, the sensing circuit, and the controller are located within the housing;
   a first lead coupled to and extending from the housing to a first distal end region;
   a second lead coupled to and extending from the housing to a second distal end region; and
   a third lead coupled to and extending from the housing to a third distal end region,
   wherein the plurality of electrodes further comprise a right ventricular electrode to deliver cardiac therapy to or sense electrical activity of the right ventricle of the patient's heart coupled to the third distal end region of the third lead, wherein the tissue-piercing electrode is coupled to the first distal end region of the first lead, wherein the right atrial electrode is coupled to the second distal end region of the second lead.
14. An implantable medical device comprising:
   a housing extending from a proximal end region to a distal end region;
   a plurality of electrodes comprising:
      a tissue-piercing electrode leadlessly coupled to the distal end region of the housing and implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart, and
      a right atrial electrode leadlessly coupled to the housing and positionable within the right atrium to deliver cardiac therapy to or sense electrical activity of the right atrium of the patient's heart;
   a therapy delivery circuit within the housing operably coupled to the plurality of electrodes to deliver cardiac therapy to the patient's heart;
   a sensing circuit within the housing operably coupled to the plurality of electrodes to sense electrical activity of the patient's heart; and
   a controller comprising processing circuitry within the housing operably coupled to the therapy delivery circuit and the sensing circuit, the controller configured to:
      monitor electrical activity of the right atrium using the right atrial electrode; and
      deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored electrical activity of the right atrium.
15. An implantable medical device comprising:
   a plurality of electrodes comprising:
      a tissue-piercing electrode implantable from the triangle of Koch region of the right atrium through the right atrial endocardium and central fibrous body to at least one of deliver cardiac therapy to and sense electrical activity of the left ventricle in the basal and/or septal region of the left ventricular myocardium of a patient's heart, and
      a right atrial motion detector positionable within the right atrium to sense mechanical activity of the right atrium of the patient's heart;
   a therapy delivery circuit operably coupled to the plurality of electrodes to deliver cardiac therapy to the patient's heart;
   a sensing circuit operably coupled to the plurality of electrodes to sense electrical activity of the patient's heart; and
   a controller comprising processing circuitry operably coupled to the therapy delivery circuit and the sensing circuit, the controller configured to:
      monitor mechanical activity of the right atrium using the right atrial motion detector; and
      deliver atrioventricular synchronous pacing using at least the tissue-piercing electrode implanted in the basal and/or septal region of the left ventricular myocardium of the patient's heart from the triangle of Koch region of the right atrium through the right atrial endocardium and central fibrous body to pace one or both ventricles based on the monitored mechanical activity of the right atrium.

## Claims

1. An implantable medical device, comprising:
a plurality of electrodes comprising:
a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart; and
a right atrial electrode positionable within the right atrium to deliver cardiac therapy to or sense electrical activity of the right atrium of the patient's heart;
a therapy delivery circuit operably coupled to the plurality of electrodes to deliver cardiac therapy to the patient's heart;
a sensing circuit operably coupled to the plurality of electrodes to sense electrical activity of the patient's heart; and
a controller including processing circuitry operably coupled to the therapy delivery circuit and the sensing circuit, the controller being configured to:
monitor electrical activity of the right atrium using the right atrial electrode;
monitor electrical activity of the left ventricle using the tissue-piercing electrode implanted in the basal and/or septal region of the left ventricular myocardium of the patient's heart from the triangle of Koch region of the right atrium through the right atrial endocardium and central fibrous body to sense electrical activity of the left ventricle in the basal and/or septal region of the left ventricular myocardium; and
deliver tachycardia therapy based on the monitored electrical activities of the right atrium and the left ventricle.

2. The device of claim 1, wherein the controller is further configured to deliver tachycardia therapy based on the monitored electrical activities of the right atrium and left ventricle including delivering at least one of anti-tachycardia pacing therapy using the plurality of electrodes and shock therapy using a separate medical device.

3. The device of claim 1 or 2, wherein the controller is further configured to withhold tachycardia therapy in response to a determining that a cardiac rhythm of the patient's heart has 1:1 atrioventricular conduction.

4. The device of any one of claims 1 to 3, further comprising a housing extending from a proximal end region to a distal end region, wherein the right atrial electrode is leadlessly coupled to the housing and the tissue-piercing electrode is leadlessly coupled to the distal end region of the housing, wherein the therapy delivery circuit, the sensing circuit, and the controller are enclosed within the housing.

5. The device of claim 4, further comprising a fixation member that extends from the housing, wherein the fixation member is configured to pierce into the myocardium and extends from the distal end region of the housing toward a distal tip of the tissue-piercing electrode.

6. The device of any one of claims 1 to 5, further comprising:
a housing, wherein the therapy delivery circuit, the sensing circuit, and the controller are located within the housing; and
a lead coupled to and extending from the housing to a distal end region, wherein the tissue-piercing electrode and the right atrial electrode are coupled to the distal end region of the lead.

7. The device of any one of claims 1 to 6, further comprising:
a housing, wherein the therapy delivery circuit, the sensing circuit, and the controller are located within the housing;
a first lead coupled to and extending from the housing to a first distal end region; and
a second lead coupled to and extending from the housing to a second distal end region,
wherein the tissue-piercing electrode is coupled to the first distal end region of the first lead, wherein the right atrial electrode is coupled to the second distal end region of the second lead.

8. The device of any one of claims 1 to 7, further comprising:
a housing, wherein the therapy delivery circuit, the sensing circuit, and the controller are located within the housing;
a first lead coupled to and extending from the housing to a first distal end region;
a second lead coupled to and extending from the housing to a second distal end region; and
a third lead coupled to and extending from the housing to a third distal end region,
wherein the plurality of electrodes further includes a right ventricular electrode to deliver cardiac therapy to or sense electrical activity of the right ventricle of the patient's heart coupled to the third distal end region of the third lead,
wherein the tissue-piercing electrode is coupled to the first distal end region of the first lead, and
wherein the right atrial electrode is coupled to the second distal end region of the second lead.

9. An implantable medical device, comprising:
a plurality of electrodes comprising:
a tissue-piercing electrode implantable from the triangle of Koch region of the right atrium through the right atrial endocardium and central fibrous body to deliver cardiac therapy to or sense electrical activity of the left ventricle in the basal and/or septal region of the left ventricular myocardium of a patient's heart; and
a right atrial electrode positionable within the right atrium to deliver cardiac therapy to or sense electrical activity of the right atrium of the patient's heart;
a therapy delivery circuit operably coupled to the plurality of electrodes to deliver cardiac therapy to the patient's heart;
a sensing circuit operably coupled to the plurality of electrodes to sense electrical activity of the patient's heart;
a controller including processing circuitry operably coupled to the therapy delivery circuit and the sensing circuit, the controller being configured to:
monitor electrical activity of the right atrium using the right atrial electrode;
monitor electrical activity of the left ventricle using the tissue-piercing electrode implanted to sense electrical activity of the left ventricle in the basal and/or septal region of the left ventricular myocardium of the patient's heart from the triangle of Koch region of the right atrium through the right atrial endocardium and central fibrous body; and
determine tachycardia of the patient's heart based on the monitored electrical activities of the right atrium and the left ventricle.

10. The device of claim 9, wherein the controller is further configured to determine whether a cardiac rhythm of the patient's heart has 1:1 atrioventricular conduction.

11. The device of claim 10, wherein the controller is further configured to determine appropriate tachycardia therapy based on the monitored electrical activities of the right atrium and the left ventricle in response to the cardiac rhythm not having 1:1 atrioventricular conduction.

12. The device of any one of claims 9 to 11, wherein determining tachycardia of the patient's heart based on the monitored electrical activities of the right atrium and the left ventricle includes determining that an atrial rhythm is regular and that a ventricular rhythm exceeds a selected ventricular rhythm threshold.

13. The device of any one of claims 9 to 12, wherein the controller is further configured to deliver tachycardia therapy in response to determining tachycardia of the patient's heart.

14. The device of claim 13, wherein the controller is further configured to deliver anti-tachycardia pacing therapy in response to a regular V-V event interval and a regular electrogram morphology.

15. The device of any one of claims 9 to 14, wherein the controller is further configured to deliver shock therapy using a separate medical device in response to at least one of an irregular V-V event interval and an irregular electrogram morphology.
